# EUROPÄISCHE PATENTSCHRIFT

(11) **EP 3 738 632 B1**
(45) Veröffentlichungstag und Bekanntmachung des Hinweises auf die Patenterteilung: **16.02.2022**
(21) Anmeldenummer: 20171293.2
(22) Anmeldetag: 21.10.2009
(51) Int. Cl.: A61M 15/06

(54) **INHALATOR**
INHALER
INHALATEUR

(30) Priorität: 23.10.2008 AT 16602008; 17.04.2009 AT 5972009
(43) Veröffentlichungstag der Anmeldung: 18.11.2020
(62) Teilanmeldung aus: 17189951.1
(73) Patentinhaber: Nicoventures Trading Limited, London WC2R 3LA (GB)
(72) Erfinder: Buchberger, Helmut, 4490 St. Florian (AT)
(74) Vertreter: Schrell, Andreas

(56) Entgegenhaltungen:
- EP-A2- 0 295 122

## Beschreibung

Die Erfindung betrifft eine Inhalatorkomponente für die Bildung eines nikotinhaltigen Dampf-Luft-Gemisches oder/und Kondensationsaerosols durch Verdampfung einer mittels Ethanol oder/und Wasser hochverdünnten Nikotinlösung, umfassend:
ein Gehäuse;
eine im Gehäuse angeordnete Kammer;
eine Lufteinlaßöffnung für die Zufuhr von Luft aus der Umgebung in die Kammer;
einen Verdampfer zur Verdampfung einer Portion der hochverdünnten Nikotinlösung mit einer in der Kammer angeordneten Verdampfungsfläche bzw. Dampfaustrittsfläche, aus welcher der erzeugte Dampf in die Kammer übertritt und sich in der Kammer mit der durch die Lufteinlaßöffnung zugeführten Luft mischt, wodurch sich schließlich das nikotinhaltige Dampf-Luft-Gemisch oder/und Kondensationsaerosol, wobei die Inhalatorkomponente eine Kondensat-Drainage- und Speichereinrichtung umfasst, wobei die Kondensat-Drainage- und Speichereinrichtung einen Schwamm umfasst.

In der gegenständlichen Patentanmeldung bezieht sich der Begriff "Inhalator" auf medizinische wie nicht-medizinische Inhalationsapparate. Der Begriff bezieht sich außerdem auf Rauchartikel und Zigarettenersatz-Artikel, wie sie beispielsweise in der Europäische Patentklasse A24F47/00B enthalten sind, soweit diese dazu bestimmt sind, dem Benutzer ein Dampf-Luft-Gemisch oder/und Kondensationsaerosol darzureichen.

Die Erfindung bezieht sich auf "Zug-Inhalatoren" bzw. auf Inhalatorkomponenten solcher Zug-Inhalatoren, welche einen intermittierenden, zugsynchronen Betrieb erlauben. Eine solche Betriebsart liegt vor, wenn die Nikotinlösung nur während eines Zuges verdampft wird, während in Intervallen zwischen zwei Zügen im Wesentlichen keine Verdampfung erfolgt. Zug-Inhalatoren sind Inhalationsapparate, bei welchen das gebildete Dampf-Luft-Gemisch oder/und Kondensationsaerosol dem Benutzer wie bei einer Zigarette in zwei Schritten zugeführt wird, nämlich zuerst als Zug in die Mundhöhle (erster Schritt) und - nach Absetzen des Inhalators - in Form einer daran anschließenden Lungeninhalation (zweiter Schritt). Das Zugvolumen beträgt dabei individuell variierend etwa 20-80mL. Im Gegensatz dazu erfolgt bei "klassischen Inhalatoren" die Zufuhr des gebildete Dampf-Luft-Gemisches oder/und Kondensationsaerosols in einem einzigen Schritt als direkte Lungeninhalation. Klassische Inhalatoren weisen im Vergleich zu Zug-Inhalatoren einen deutlich höheren Luftdurchsatz durch den Inhalator auf: ca. 100-750mL/s gegenüber 10-40mL/s. Der vergleichsweise geringe Luftdurchsatz führt bei Zug-Inhalatoren im Zusammenwirken mit einem hohen ethanolischen oder/und wässrigen Lösungsmittelanteil in der Nikotinlösung zu einer Reihe von Problemen, welche später noch im Detail erörtert werden.

Als eine "mittels Ethanol oder/und Wasser hochverdünnte Nikotinlösung" gelte im Rahmen der gegenständlichen Patentanmeldung jede nikotinhaltige Lösung, deren Massenanteil an Ethanol oder/und Wasser zusammen mindestens 50% beträgt. Ein hoher ethanolischer oder/und wässriger Lösungsmittelanteil in der Nikotinlösung hat eine Reihe von vorteilhaften Effekten. WO 03/101454 (Mark Warchol et al.) beschreibt beispielhaft solche hochverdünnten Nikotinlösungen, konkret gepufferte bzw. pH-regulierte Nikotinlösungen. Als Lösungsmittel kommen danach primär Ethanol oder/und Wasser zum Einsatz, deren Massenanteil in der Nikotinlösung mindestens 50%, vorzugsweise mindestens 90% und besonders bevorzugt um 99% beträgt. WO 03/101454 beschreibt ferner die zusätzliche Beimischung von Propylenglykol, Glycerol oder/und Polyethylenglykol zur hochverdünnten Nikotinlösung. Diese Substanzen haben neben einer grundsätzlich lösungsvermittelnden Wirkung auch eine aerosolbildende Wirkung. Bezüglich des Nikotins wirken diese Substanzen außerdem maskierend, indem sie das organoleptische Reizpotenzial des Nikotins durch Verdünnung dämpfen. Schließlich sind in WO 03/101454 verschiedene organische und anorganische Säuren zur Regulierung des pH-Wertes der Nikotinlösung aufgeführt.

Obwohl die WO 03/101454 als Mittel zur Aerosolbildung vorzugsweise Vernebler basierend auf Ultraschall-, Druckluft-, oder elektrohydrodynamischen Zerstäubungsprinzipien vorschlägt, lassen sich solche hochverdünnten Nikotinlösungen, wie eigene umfangreiche Versuchsreihen zeigten, mit Einschränkungen auch nach dem eingangs beschriebenen Verdampfungs- und Kondensationsprinzip in ein inhalierbares Aerosol überführen. Eine solche Einschränkung ist, daß die Nikotinlösung nur rückstandsfrei verdampfende Inhaltsstoffe enthält, vor allem, wenn der Verdampfer wiederholt verwendet werden soll. Hier zeigt sich bereits eine erste vorteilhafte Wirkung des hohen Anteils an Ethanol oder/und Wasser, welche nämlich durch ihren niedrigen Siedepunkt die Verdampfbarkeit der Nikotinlösung deutlich verbessern. Auf diese Weise können auch ansich - das heißt für sich alleine gesehen - nicht rückstandsfrei verdampfende Inhaltsstoffe weitgehend rückstandsfrei in das Kondensationsaerosol übergeführt werden. Als Beispiel sei Milchsäure angeführt, welche als pH-regulierendes Mittel in der Nikotinlösung Verwendung finden kann: als Einzelsubstanz verdampft hinterläßt Milchsäure auf der Verdampfungsfläche stets Verdampfungsrückstände. Erfolgt die Verdampfung der Milchsäure in einer mit Ethanol oder/und Wasser hochverdünnten Lösung, bleiben praktisch keine Verdampfungsrückstände zurück. Die Verbesserung der Verdampfbarkeit geht Hand in Hand mit einer Verringerung der thermischen Zersetzung von weniger stabilen Inhaltsstoffen in der hochverdünnten Nikotinlösung. Davon profitiert auch das Nikotin selbst (Siedepunkt: 246°C), welches als Einzelsubstanz verdampft eine gewisse Tendenz zur thermischen Zersetzung zeigt.

Weitere vorteilhafte Wirkungen des hohen Anteils an Ethanol oder/und Wasser in der Nikotinlösung sind: Verbesserung der Dosierbarkeit des eigentlichen Wirkstoffes Nikotin, allgemeine Verbesserung der Löslichkeit für weitere Inhaltsstoffe in der Nikotinlösung, Verbesserung verschiedener physikalischer Eigenschaften, wie beispielsweise eine Verringerung der Viskosität der Nikotinlösung. Letzterer Effekt kommt vor allem zum Tragen, wenn der Transport der Nikotinlösung zum Verdampfer oder Zerstäuber mittels eines Dochts erfolgt. Wasser vermindert zudem die Hygroskopizität des gebildeten Aerosols und beugt so einem Austrocknen der Mundhöhle und des Rachens vor. Ethanol und Wasser wirken zusammen ferner keimtötend und vebessern dadurch die Hygiene der Aerosolerzeugung und -verabreichung.

Mittels Ethanol oder/und Wasser hochverdünnte Nikotinlösungen, wie sie beispielhaft in WO 03/101454 vorgeschlagen werden, führen bei Verwendung derselben in Inhalatorkomponenten von Zug-Inhalatoren, wie eingangs beschrieben, zu einer Reihe von Problemen: der hohe Lösungsmittelbalast führt einmal dazu, daß, gemessen an der eigentlich zugeführten Nikotinmenge bzw. - dosis, vergleichsweise große Dampfmengen an Ethanol oder/und Wasser in der Kammer freigesetzt werden, welche wegen ihres hohen Dampfdruckes und der geringen durchgesetzten Luftmenge (etwa 20-80mL) erhebliche Mengen an Kondensatrückständen nach sich ziehen. Die Kondensatrückstände scheiden sich vor allem an der Kammerinnenwand und an anderen Strukturkomponenten in der Kammer und stromabwärts von der Kammer ab. Die Menge an gebildeten Kondensatrückständen hängt sekundär von weiteren Faktoren ab, insbesondere von den spezifischen Strömungsverhältnissen in der Kammer und der Mischungsdynamik zwischen dem Dampf und der Luft. Die abgeschiedenen Kondensatrückstände können sich zu frei beweglichen Kondensatansammlungen vereinigen, welche die Funktion der Inhalatorkomponente stören können. Die Kondensatansammlungen können aber auch über die Mundstücköffnung in die Mundhöhle des Benutzers gelangen und dort das Geschmacksempfinden des Benutzers stören. Zudem enthalten solche Kondensatansammlungen stets auch gewisse Mengen an Nikotinkondensat, welches, wenn es schubweise zusammen mit einer größeren Kondensatansammlung in die Mundhöhle gerät, auch gesundheitsschädliche und toxische Wirkungen entfalten kann. In eigenen umfangreichen Versuchsreihen zeigte sich ferner unerwartet, daß der Genuß von auf Basis solcher hochverdünnter Nikotinlösungen in Zug-Inhalatoren erzeugten Kondensationsaerosolen mit nachteiligen organoleptischen Wirkungen, unter anderem mit einem störenden Geschmacksreiz auf der Zungenspitze und den Lippen verbunden ist. Die Ursache dafür hängt auf jeden Fall mit dem großen Ethanol- oder/und Wasseranteil in der Nikotinlösung zusammen; die genauen Wirkmechanismen lagen jedoch zu diesem Zeitpunkt noch im Dunkeln.

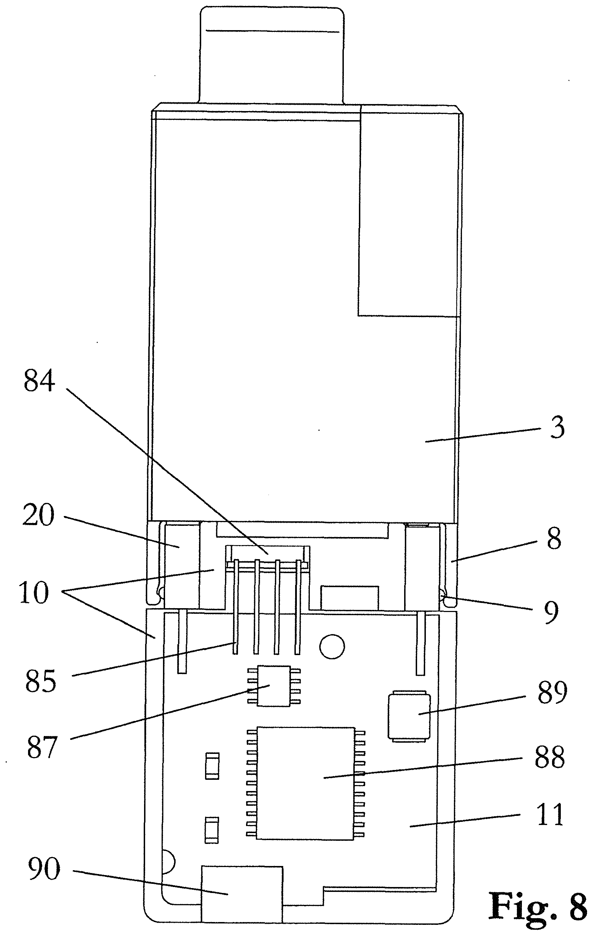

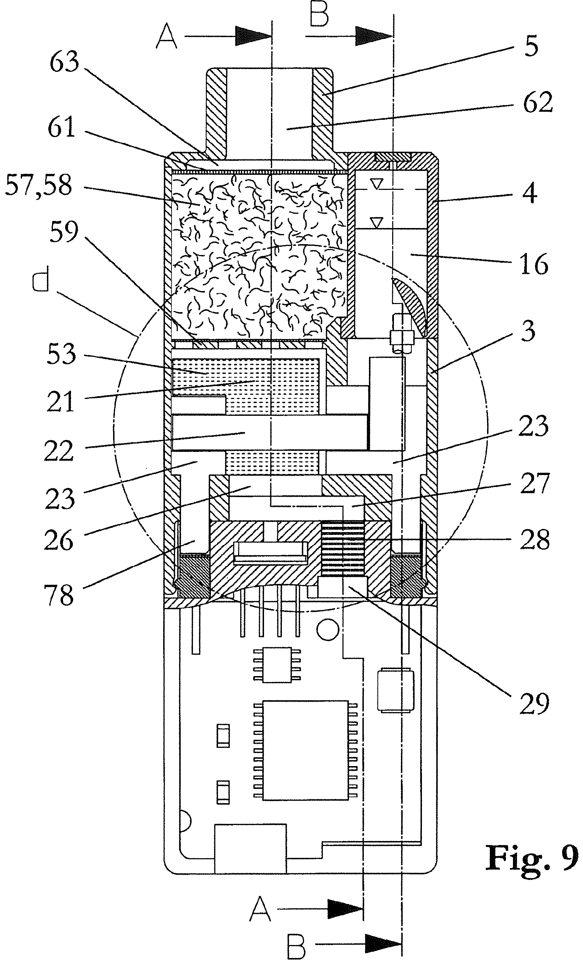

US 6,155,268 (Manabu Takeuchi) beschreibt ein Aroma-erzeugendes Gerät, bestehend aus (Fig. 8) einem Flüssigkeitsbehälter 32 zur Aufnahme eines flüssigen Aromamittels 34, eine durch zwei Platten 361, 362 gebildete Kapillare 371, welche mit dem flüssigen Aromamittel 34 kommuniziert und in einem Endabschitt mittels zweier plattenförmiger Heizelemente 421, 422 aufgeheizt werden kann. Das flüssige Aromamittel 34 strömt durch die in der Kapillare 371 wirkenden Kapillarkräfte zum Heizelement 421, 422, wo es verdampft und als Dampfstrom in eine Kammer 121 strömt, welche zumindest teilweise durch ein Mundstück 161 gebildet wird. Das Aroma-erzeugende Gerät ist insoweit vom Aufbau her den sogenannten Kapillarrohr-Aerosol-Generatoren zuzuordnen. Im Mundstück kann ferner optional ein Filter 102 vorgesehen sein. Die Platten 361, 362 und plattenförmigen Heizelemente 421, 422 sind außen von einem zylindrischen Körper 103 umgeben, wobei ein Ringspalt 104 gebildet wird (siehe auch Fig. 9). Der Ringspalt 104 und die Kapillare 371 sind so ausgerichtet, daß sie zur Austrittsöffnung des Mundstücks 161 weisen. Die während einer Inhalation oder eines Zuges angesaugte Luft tritt von außen durch die Öffnung 18 in das Gerät ein und gelangt in den Ringspalt 104. Der aus der Kapillare 371 austretende Dampf wird durch die aus dem Ringspalt 104 austretende Luft ummantelt und zur Austrittsöffnung des Mundstücks 161 geführt. Auf diese Weise soll vermieden werden, daß sich Teile des gebildeten Dampfes als Kondensat auf den Innenflächen der Kammer 121 ablagern. Sollte es doch zu Ablagerungen kommen, so setzen sich diese vornehmlich auf der inneren Oberfläche des auswechselbaren Mundstücks 161 ab, da dieses nämlich einen Großteil der Kammer 121 auskleidet und sich fast bis zur Mündung der Kapillare 371 erstreckt.

Das flüssige Aromamittel 34 beinhaltet zumindest einen Aromastoff. Zusätzlich können noch aerosolbildende Substanzen enthalten sein, und zwar Alkohole, Zucker, Wasser sowie eine Mischung aus zumindest zwei dieser Substanzen. Als Alkohole können beispielsweise Glycerin, Propylenglykol sowie Mischungen derselben Verwendung finden. Im Fall, daß das Aroma-erzeugende Gerät als simulierender Rauchartikel (Zigarettenersatz) verwendet werden soll, kann das flüssige Aromamittel 34 auch Tabakkomponenten wie Tabakextrakte und Tabakrauchkondensat enthalten. Die Verwendung von Nikotin als Inhaltsstoff wird explizit nicht erwähnt, allerdings enthalten Tabakextrakte und insbesondere Tabakrauchkondensat in der Regel Nikotin.

Grundsätzlich muß angemerkt werden, daß der Prozess der Bildung von Kondensatablagerungen in erster Linie durch molekulare Diffusionsvorgänge in der Dampfphase bestimmt wird und insofern von makroskopischen Strömungsbedingungen nur begrenzt beeinflußt werden kann. Bestünde das flüssige Aromamittel 34 aus einer mittels Ethanol oder/und Wasser hochverdünnten Nikotinlösung, und setzt man voraus, daß das Aroma-erzeugende Gerät nach dem Prinzip eines Zug-Inhalators arbeitet und zugsynchron betrieben wird, so wäre trotz der ergriffenen strömungstechnischen Optimierungsmaßnahmen mit erheblichen Diffusionsströmen in Richtung der Innenfläche der Kammer 121 bzw. des Mundstücks 161 und damit verbundenen mit erheblichen Kondensatabscheidungen zu rechnen. Die Aufnahmekapazität der Innenflächen der Kammer 121 und des Mundstücks 161 für Kondensatrückstände wäre jedenfalls schnell erschöpft. Würde das Mundstück 161 nicht in kurzen Abständen gewechselt werden, bildeten sich bald frei bewegliche Kondensatansammlungen, welche die Funktion des Aroma-erzeugenden Gerätes stören könnten. Aber auch schon allein die Möglichkeit an sich, das Kondensat vom Benutzer selbst aus dem Gerät entfernen zu lassen, birgt ein Risiko für die Umwelt, weil davon auszugehen ist, daß das Kondensat auch Nikotinreste enthält. Die Kondensatansammlungen im Inneren der Kammer 121 könnten aber auch über die Mundstücköffnung in die Mundhöhle des Benutzers gelangen, verbunden mit den bereits erwähnten Nachteilen und Gefahren. Im Fall, daß im Mundstück 161 der Filter 102 appliziert ist, könnten die Kondensatansammlungen den Filter 102 blockieren, wodurch der Strömungswiderstand sprunghaft ansteigen würde. Der Filter 102, welcher als Option vorgesehen ist, dient offenbar nur dazu, einen für den Benutzer komfortablen Strömungswiderstand einzustellen.

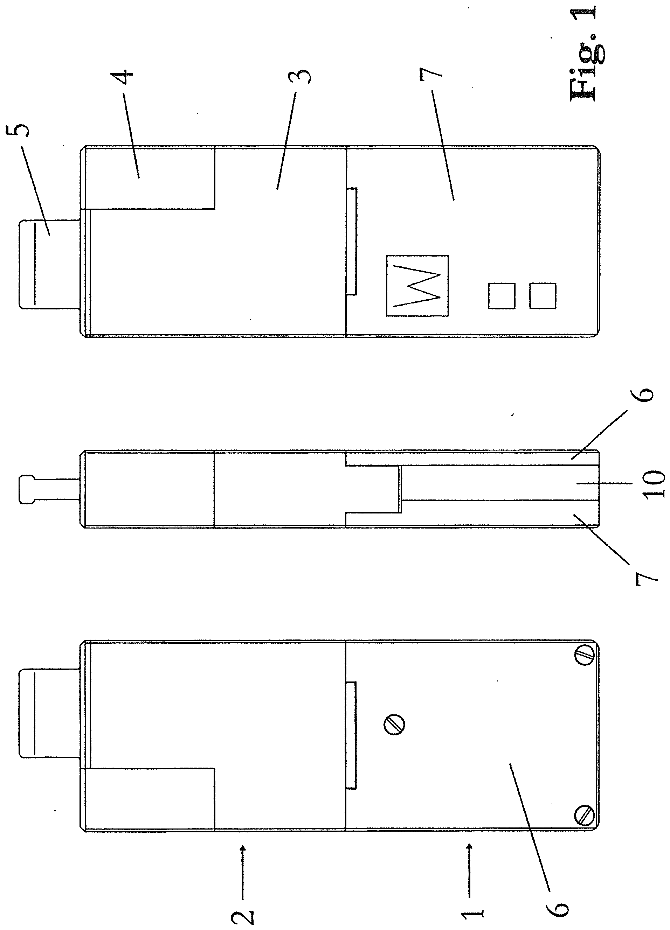

US 5,666,977 (Charles D. Higgins et al.) beschreibt einen elektrischen Rauchartikel 10 für die Erzeugung und Verabreichung von Tabakaromen oder/und Tabakaerosolen bestehend aus (Fig. 1-3) einem wiederverwendbaren Teil 20 und einem auswechselbaren Filtereinsatz 21, welcher in den wiederverwendbaren Teil 20 eingeschoben ist und über welchen der Benutzer die Tabakaromen oder das gebildete Aerosol bezieht. Der wiederverwendbare Teil 20 beinhaltet ein Beschickungssystem 32, 33 für die Zufuhr von flüssigem Tabakaroma zu einem Heizelement 23, eine Energiequelle 22 für die Anspeisung des Heizelements 23 mit elektrischem Strom, einen Steuerkreis 24 für diverse Steueraufgaben und schließlich eine Patrone 28 für die Aufnahme des flüssigen Tabakaromas. Der auswechselbare Filtereinsatz 21 hat neben dem Zweck der Filterung die Aufgabe, einen für den Benutzer komfortablen Strömungswiderstand herzustellen. Der auswechselbare Filtereinsatz 21 enthält außerdem einen Wandteil 21A, welcher das Heizelement 23 und das Beschickungssystem 32, 33 weitgehend umschließt. Dieser Wandteil 21A bildet insofern eine Barriere zu permanenten Strukturkomponenten des wiederverwendbaren Teils 20, wodurch die Gefahr von Kondensatablagerungen auf diesen Strukturkomponenten reduziert wird.

Das flüssige Tabakaroma kann laut US 5,666,977 aus jedem beliebigen flüssigen Material bestehen, solange bei Erhitzung desselben Tabakaromen freigesetzt werden. Das Tabakaroma kann Tabakauszüge beinhalten oder auch nicht. Ferner ist es wünschenswert, daß das flüssige Tabakaroma ein aerosolbildendes Material beinhaltet. Die Verwendung von Nikotin als Inhaltsstoff wird explizit nicht erwähnt, allerdings enthalten Tabakauszüge in der Regel Nikotin. Fest steht, daß die Aufnahmekapazität der Innenfläche des Wandteils 21A für Kondensat limitiert ist. Bestünde das flüssige Tabakaroma aus einer mittels Ethanol oder/und Wasser hochverdünnten Nikotinlösung, würden sich ähnliche Probleme ergeben, wie sie bereits zuvor bei der Erörterung der US 6,155,268 dargestellt wurden.

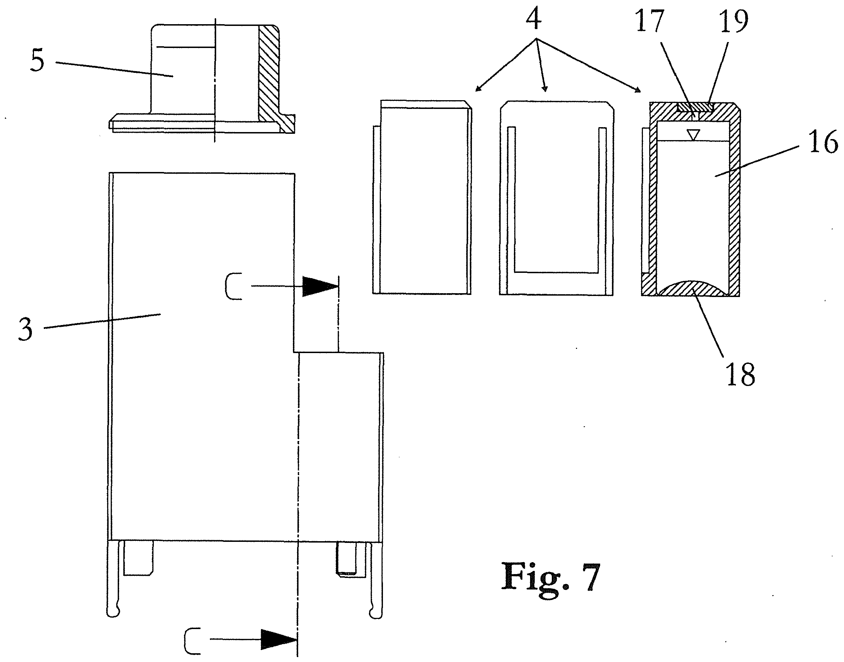

US 5,390,864 und US 5,553,791 (Dennis R. Alexander) beschreiben eine Vorrichtung für die Zerstäubung und Verabreichung von medizinischen Lösungen mittels Laser. Nach Fig. 7-9 besteht die Vorrichtung aus einer Sonde 182 die bis zur Lunge eines Patienten 180 vorgeschoben wird, an deren Ende eine Massen-Steuereinheit 26E für die Verteilung des gebildeten Aerosols angeordnet ist. Fig. 9 zeigt den Aufbau der Massen-Steuereinheit 26E: etwa im Zentrum der Sonde 182 verläuft der Lichtleiter 188, welcher von einem Laser 52E (Fig. 7) gespeist wird. Der Lichtleiter 188 wird koaxial vom Medikationsschlauch 190 umgeben, welcher an seinem Ende eine Öffnung 197 bildet. Die Öffnung 197 hat vorzugsweise einen Durchmesser von etwa 100µm. Am Ende des Lichtleiters 188 und unmittelbar vor der Öffnung 197 befindet sich die Fokusierlinse 199, welche vom aus dem Lichtleiter 188 austretenden Licht beaufschlagt wird, und den Lichtstrahl derart fokusiert, daß die medizinische Lösung zerstäubt wird, ohne das umgebende Gewebe dabei zu gefährden. Die auf diese Weise erzeugten Aerosolpartikel 185 verlassen die Sonde 182 über die Öffnung 194. Die Öffnung 194 wird durch gekrümmte bzw. becherförmige Begrenzungswände 183 gebildet und befindet sich in einer vertikal erhöhten Position. Durch diese Anordnung wird erreicht, daß im Zuge der Zerstäubung gebildete größere Tropfen an den Begrenzungswänden 183 abgeschieden werden, und nur relativ feine Partikel einheitlicher Größe die Öffnung 194 passieren können. Die Begrenzungswände 183 bzw. die Innenwand der Sonde 182 ist mit einem absorbierenden Material 192 ausgekleidet. Das absorbierende Material 192 dient zur Aufnahme von unzerstäubten Rückständen der medizinischen Lösung und besteht beispielsweise aus Papier oder aus einem Kunststoffschwamm. Zur Unterstützung der Zerstäubung kann über eine Kapillarleitung 201 zusätzlich Gas, beispielsweise Luft in die Aerosolbildungszone eingespeist werden.

Die Anordnung nach US 5,390,864 und US 5,553,791 betrifft keinen Inhalator im üblichen Sinne. Vielmehr wird das Medikament über eine Sonde direkt in der Lunge appliziert. Durch die zuvor beschriebenen konstruktiven Maßnahmen soll vermieden werden, daß unzerstäubte Teile des verabreichten Medikaments in die Lunge des Patienten gelangen. Die der gegenständlichen Anmeldung zugrundeliegende Problematik im Zusammenhang mit dem hohen Ethanol-oder/und Wasserbalast und den damit verbundenen nachteiligen organoleptischen Wirkungen, kann bei der Lungenapplikation nach den Fig. 7-9 gar nicht erst in Erscheinung treten. Die US 5,390,864 und US 5,553,791 geben auch keinerlei Lehre in diese Richtung.

Eine weitere Inhalatorkomponente zur Bildung eines nikotinhaltigen Dampf-Luft Gemisches ist aus EP0295122A2 bekannt.

Die Erfindung wird in dem unabhängigen Anspruch 1 definiert. Bevorzugte Ausführungsformen bilden den Gegenstand der abhängigen Ansprüche.

Der Erfindung liegt die Aufgabe zugrunde, die zuvor aufgezeigten Nachteile zu beheben. Der Erfindung liegt insbesondere die Aufgabe zugrunde, eine Inhalatorkomponente der eingangs geschilderten Art so auszugestalten, daß der hohe Ethanol- oder/und Wasseranteil in der zu verdampfenden Nikotinlösung und in der Folge im gebildeten Dampf-Luft-Gemisch bzw. Kondensationsaerosol sich weder auf die Funktion der Inhalatorkomponente noch auf den Benutzer oder dessen Umwelt nachteilig auswirkt. Konkret soll verhindert werden, daß sich im Inneren der Inhalatorkomponente über die gesamte Nutzungsdauer der Inhalatorkomponente hinweg frei bewegliche Kondensatansammlungen ablagern, welche in die Mundhöhle des Benutzers gelangen oder die Funktion der Inhalatorkomponente stören könnten. Kondensatrückstände sollen auch nicht auf anderem Wege in die Umwelt gelangen können. Ferner soll das gebildete Dampf-Luft-Gemisch bzw. Kondensationsaerosol vom Benutzer ohne nachteilige organoleptische Nebenwirkungen zu konsumieren sein. Insbesondere bedeutet dies, daß der Genuß des gebildeten nikotinhaltigen Dampf-Luft-Gemisches bzw. Kondensationsaerosols auf der Zungenspitze, auf den Lippen und in der Mundhöhle des Benutzers keine unangenehmen oder störenden Geschmacksempfindungen provozieren darf und gut inhalierbar sein soll, wobei vorausgesetzt wird, daß die verabreichte Nikotindosis mit jener von Zigaretten vergleichbar ist, also bis zu 0,2mg pro Zug betragen kann. Anders formuliert soll ausgehend von einer Inhalatorkomponente der eingangs geschilderten Art ein möglichst vollwertiger Zigarettenersatz geschaffen werden, und zwar sowohl hinsichtlich der pharmakologischen und pharmakokinetischen Wirkungen als auch hinsichtlich der organoleptischen Wirkungen des erzeugten Dampf-Luft-Gemisches bzw. Kondensationsaerosols. Die Inhalatorkomponente soll schließlich ergonomisch zu handhaben sein und möglichst kompakt und raumsparend ausgestaltet werden, so daß eine Verwendung als Tascheninhalator möglich ist.

Die Inhalatorkomponente kann eine zweistufige Lösungsmittel-Abscheidevorrichtung aufweisen, bestehend erstens aus einer mit der Kammer kommunizierenden Kondensat-Drainage- und Speichereinrichtung und zweitens aus einem vom gebildeten Dampf-Luft-Gemisch oder/und Kondensationsaerosol durchströmbaren Kühler. Die zweistufige Lösungsmittel-Abscheidevorrichtung hat den Effekt, daß der im gebildeten Dampf-Luft-Gemisch bzw. Kondensationsaerosol enthaltene erhebliche Ethanol- oder/und Wasserdampfbalast sehr weitgehend auskondensiert und abgeschieden wird, ohne daß hierbei die Funktion der Inhalatorkomponente gestört würde. Die Kondensat-Drainage- und Speichereinrichtung, welche die erste Stufe der Lösungsmittel-Abscheidevorrichtung darstellt, dient dazu, die in der Kammer in großer Menge anfallenden Kondensatrückstände aufzunehmen, abzuleiten und zu speichern. Die Kondensatrückstände sind eine Folge der hohen Dampfkonzentration in der Kammer, insbesondere in der Nähe der Verdampfungsfläche bzw. Dampfaustrittsfläche. Ihre Bildung wird im Wesentlichen durch eine Diffusion in Richtung kühlerer Begrenzungswände bestimmt und erfolgt blitzartig. Die Kondensat-Drainage- und Speichereinrichtung verhindert, daß sich in der Kammer freibewegliche Kondensatansammlungen bilden. Solche Kondensatansammlungen könnten nämlich auch den nachgeordneten Kühler kontaminieren und dessen Funktion beeinträchtigen. Die Kondensat-Drainage- und Speichereinrichtung kann beispielsweise aus einem oder mehreren Drainage-Kanälen bestehen, welche mit der Kammer kommunizieren und die in der Kammer gebildeten Kondensatrückstände zu einem separaten Sammelbehälter ableiten. Die Ableitung kann durch die Schwerkraft oder/und kapillar, beispielsweise mittels eines Dochts erfolgen. Der Sammelbehälter kann optional einen Schwamm (siehe nachstehende Begriffserläuterung) beinhalten, wodurch das Kondensat kapillar gebunden wird. Im Fall, daß die Ableitung des Kondensats mittels eines Dochts erfolgt, kann auf einen separaten Sammelbehälter auch verzichtet werden, nämlich wenn der Docht selbst die Funktion der Kondensatspeicherung übernimmt.

Im Kühler, welcher die zweite Stufe der Lösungsmittel-Abscheidevorrichtung darstellt, wird das Dampf-Luft-Gemisch bzw. Kondensationsaerosol gekühlt. Es zeigte sich überraschend, daß durch die Kühlung die eingangs beschriebene Problematik betreffend die mit dem hohen Ethanol- oder/und Wasserbalast zusammenhängenden nachteiligen organoleptischen Wirkungen weitgehend entschärft werden kann. Damit konnte nun auch ein Erklärungsversuch für die involvierten Wirkmechanismen unternommen werden: die Verdampfung einer mittels Ethanol oder/und Wasser hochverdünnten Nikotinlösung macht, gemessen an der eigentlich verabreichten Nikotinmenge bzw. -dosis, einen vergleichsweise großen Energiebetrag erforderlich. Dies gilt im besonderen Maße für hohe Wasseranteile, wegen der vergleichsweise hohen spezifischen Wärmekapazität und Verdampfungsenthalpie des Wassers. Ein wesentlicher Teil der im Zuge der Verdampfung zugeführten Wärme findet sich als thermische oder innere Energie im gebildeten Kondensationsaerosol wieder und manifestiert sich in einer vergleichsweise hohen Temperatur desselben. Mit zunehmender Temperatur steigt aber auch der Sättigungsdampfdruck des enthaltenen Ethanols oder/und Wassers sowie gegebenenfalls weiterer Inhaltsstoffe, insbesondere aerosolbildender Substanzen, deutlich an, was dazu führt, daß deren Konzentration in der Gasphase relativ hoch ist. Es wird ferner angenommen, daß die Flüchtigkeit von in der Partikelphase enthaltenen weiteren Substanzen, wie zum Beispiel aerosolbildenden Substanzen, durch den großen Ethanol- oder/und Wasseranteil deutlich erhöht wird. Diese Bedingungen bewirken relativ hohe Kondensationsflüsse in Richtung kühlerer Begrenzungswände und sonstiger beaufschlagter Strukturelemente. Die hohe Kondensationsrate setzt sich offenbar auch nach Übertreten des Dampf-Luft-Gemisches bzw. Kondensationsaerosols in die Mundhöhle des Benutzers fort, und scheint dort die nachteiligen organoleptischen Wirkungen zu provozieren. Die Kühlung bewirkt eine Herabsetzung der Sättigungsgrenze des im Dampf-Luft-Gemisch bzw. Kondensationsaerosol enthaltenen Dampfes und damit verbunden ein weitgehendes Auskondensieren desselben. Das Kondensat kann aus dem Dampf-Luft-Gemisch bzw. Kondensationsaerosol im Kühler abgeschieden werden, oder sich auf den Partikeln des Kondensationsaerosols niederschlagen und auf diese Weise die Partikelphase anreichern.

Begriffserläuterungen :
"Kammer": soll auch Kanäle mit einschließen; somit fällt auch ein rohrförmiger Kanal unter den Begriff "Kammer"; ein offenes Rohrende könnte in diesem Fall beispielsweise die Lufteinlaßöffnung bilden.

"Verdampfungsfläche bzw. Dampfaustrittsfläche": jene Fläche, auf welcher die Verdampfung der Nikotinlösung stattfindet, bzw. jene Fläche oder Öffnung, aus welcher der gebildete Dampf ausströmt. Im Fall von Kapillarrohr-Aerosol-Generatoren entspricht das freie Kapillarrohrende der Dampfaustrittsfläche.

Erfindungsgemäß besteht die Kondensat-Drainage- und Speichereinrichtung aus einem in der Kammer angeordneten Schwamm. Durch diese Anordnung gelingt es, die in der Kammer anfallenden Kondensatrückstände auf konstruktiv einfache und effektive Weise zu beherrschen. Der Schwamm nimmt die Kondensatrückstände an seiner äußeren Oberfläche auf und leitet sie über seine offene Kapillarstruktur nach innen hin ab. Die Kondensatrückstände werden am Ort ihres Entstehens, nämlich in der Kammer, aufgenommen, abgeleitet und gespeichert. Die Poren des Schwamms werden weder von der in die Kammer einströmenden Luft noch vom gebildeten Dampf-Luft-Gemisch bzw. Kondensationsaerosol durchströmt. Die Poren dienen auch nicht dazu, die zu verdampfende Nikotinlösung zu speichern oder zum Verdampfer zu fördern. Die Poren des Schwamms dienen ausschließlich dem Zweck, Kondensatrückstände kapillar zu binden. Zusätzliche DrainageKanäle, Dochte oder separate Sammelbehälter erübrigen sich. Die Drainage und Speicherung des Kondensats mittels eines Schwamms funktioniert auch ohne die unterstützende Wirkung der Schwerkraft und ist damit lageunabhängig. Unter den Begriff "Schwamm" falle jeder offenzellige Porenkörper, sofern dieser zumindest auf Ethanol oder/und Wasser adsorbierend/ saugfähig wirkt.

In einer vorteilhaften Weiterbildung der Erfindung füllt der Schwamm den überwiegenden Teil der Kammer aus. Auf diese Weise kann bei kompakter Bauweise eine große Aufnahmekapazität für die flüssigen Kondensatrückstände realisiert werden. Günstig ist ferner, wenn der Schwamm aus einem formbeständigen Material besteht, welches auch nach vollständiger Infiltration mit den Kondensatrückständen seine Form weitgehend beibehält. Die Formbeständigkeit gewährleistet, daß die Strömungsverhältnisse in der Kammer, und damit die Bedingungen für die Bildung des Dampf-Luft-Gemisches bzw. Kondensationsaerosols konstant bleiben. Um festzustellen, ob ein konkretes Material formbeständig ist, reicht es aus, dieses mit einer Ethanol-Wasser-Lösung zu tränken, und nach drei Tagen Verweildauer die Formbeständigkeit zu überprüfen. Der Schwamm kann aus einem festen schaumartigen Material wie Metallschaum oder Keramikschaum, aus einem porösen Sinterformkörper, aus einem porösen Füll- oder Schüttmaterial ohne Blähneigung, beispielsweise aus einer Trocknungsmittel-Granulat-Schüttung, oder aus einem porösen Faserverbund, beispielsweise gebildet aus thermisch oder mit Hilfe eines Bindemittels miteinander verbundenen Natur- oder Chemiefasern, bestehen. Wesentlich ist außerdem, daß das Material gegenüber den Kondensatrückständen weitestgehend chemisch inert ist.

Es wird ferner als erfindungsgemäß angesehen, daß der Schwamm vom Gehäuse weitgehend umschlossen wird und mit dem Gehäuse untrennbar verbunden ist. Damit soll erreicht werden, daß der Schwamm nicht direkt mit der Umwelt in Kontakt kommen kann, und eine Entfernung desselben aus dem Gehäuse nur durch Gewalteinwirkung und Zerstörung des Gehäuses und damit der Inhalatorkomponente möglich ist. Diese Schutzmaßnahme erweist sich als vorteilhaft, da das im Schwamm gebundene Kondensat stets auch Nikotinrückstände enthält, welche eine Gefahr für die Umwelt darstellen. Die Inhalatorkomponente bildet zusammen mit dem Schwamm einen Einwegartikel, welcher nach Erreichen der vorgesehenen Lebensdauer umweltgerecht zu entsorgen ist. Einzelbestandteile der Inhalatorkomponente einschließlich des Schwamms können gegebenenfalls im Zuge eines Recyclings wiederverwendet werden.

Beispielsweise ist der Kühler als Flüssigkeitskühler ausgebildet. Die Kühlflüssigkeit des Flüssigkeitskühlers wird vom gebildeten Dampf-Luft-Gemisch bzw. Kondensationsaerosol in Form von Gasblasen durchsetzt. Die treibende Kraft ist die Auftriebskraft der Gasblasen in der Kühlflüssigkeit. Der Flüssigkeitskühler kann beispielsweise vom Prinzip her ähnlich einer Wasserpfeife oder Gaswaschflasche ausgebildet sein. Die Einleitung des Dampf-Luft-Gemisches bzw. Kondensationsaerosols in die Flüssigkeit erfolgt vorteilhafterweise über eine oder mehrere Düsenöffnungen oder eine Fritte. Da das Zugvolumen der erfindungsgemäßen Inhalatorkomponente wesentlich kleiner ist als das durch eine Wasserpfeife durchgesetzte Luftvolumen (20-80mL versus 0,5-1,0L), reicht schon eine vergleichsweise kleine Kühlflüssigkeitsmenge aus, um eine ausreichende Kühlung zu bewirken. Die Kühlflüssigkeit besteht vorzugsweise aus Wasser oder/und Ethanol. Ethanol zeichnet sich unter anderem durch eine keimtötende und lösungsvermittelnde Wirkung aus. Zusätzlich können in der Kühlflüssigkeit Konservierungsmittel wie Carbonsäuren, z.B. Sorbinsäure oder Benzoesäure, enthalten sein. Durch die Zugabe von Carbonsäuren kann die Kühlflüssigkeit ferner soweit angesäuert werden, daß sie in der Lage ist, aus dem Dampf-Luft-Gemisch bzw. Kondensationsaerosol freies, unprotonisiertes Nikotin aufzunehmen und zu binden. Das freie, unprotonisierte Nikotin hat nachteilige organoleptische Eigenschaften und wirkt sich negativ auf die Inhalierbarkeit des dargebotenen Dampf-Luft-Gemisches bzw. Kondensationsaerosols aus.

Die Inhalatorkomponente umfaßt optionall eine von einem Mundstück gebildete Mundstücköffnung, welche mit der Kammer kommuniziert, und durch welche ein Benutzer das nikotinhaltige Dampf-Luft-Gemisch oder/und Kondensationsaerosol dargeboten erhält, und es ist vorgesehen, daß der Flüssigkeitskühler mit der Kammer einerseits und mit der Mundstücköffnung andererseits jeweils über eine Flüssigkeitssperre in Form eines offenporigen Porenkörpers oder einer semipermeablen Membran mit hydrophoben Materialeigenschaften kommuniziert. Der offenporige Porenkörper bzw. die semipermeable Membran ist für das Dampf-Luft-Gemisch bzw. Kondensationsaerosol durchlässig; seine hydrophoben Materialeigenschaften verhindern jedoch, daß Kühlflüssigkeit in die Kammer gelangt oder über die Mundstücköffnung austritt, und zwar unabhängig von der räumlichen Lage der Inhalatorkomponente. Als hydrophobes Material eignet sich grundsätzlich jedes von Natur aus hydrophobe Material sowie durch geeignete Verfahren hydrophobisiertes Material. Als typisches Beispiel für ein naturhydrophobes Material sei PTFE (Polytetrafluorethylen) genannt. In einer vorteilhaften Ausgestaltung umfaßt die Inhalatorkomponente einen Düsenkörper zur Einleitung des in der Kammer gebildeten Dampf-Luft-Gemisches bzw. Kondensationsaerosols in die Kühlflüssigkeit des Flüssigkeitskühlers, und es ist vorgesehen, daß der Düsenkörper durch den offenporigen Porenkörper bzw. durch die semipermeable Membran gebildet wird. Der offenporige Porenkörper bzw. die semipermeable Membran erfüllt in diesem Fall also zwei Aufgaben gleichzeitig, nämlich erstens die Verdüsung des Dampf-Luft-Gemisches bzw. Kondensationsaerosols in der Kühlflüssigkeit, und zweitens die Verhinderung eines Ausfließens der Kühlflüssigkeit in die Kammer.

In einer Weiterbildung umfaßt die Inhalatorkomponente ein Mundstück, über welches ein Benutzer das nikotinhaltige Dampf-Luft-Gemisch oder/und Kondensationsaerosol dargeboten erhält, sowie ein die Kühlflüssigkeit des Flüssigkeitskühlers aufnehmendes Flachgehäuse, und es ist vorgesehen, daß das Mundstück an einer Stirnfläche des Flachgehäuses ansetzt und aus der Stirnfläche gekrümmt vorspringt, ohne über eine der Begrenzungsebenen des Flachgehäuses hinauszuragen. Diese Anordnung des Mundstücks ermöglicht es dem Benutzer, das Flachgehäuse während eines Zuges in annähernd vertikaler Lage zu halten, wenn man eine aufrechte oder leicht geneigte Kopfhaltung des Benutzers voraussetzt. Die vertikale Lage gewährleistet, daß die Kühlflüssigkeit allseits einen annähernd konstanten Flüssigkeitspegel aufweist, und die Gasblasen die Kühlflüssigkeit über den gesamten Querschnitt relativ gleichmäßig durchsetzen. Dadurch, daß das Mundstück innerhalb der Begrenzungsebenen des Flachgehäuses angeordnet ist, liegt das Flachgehäuse nach dem Ablegen der Inhalatorkomponente auf einer ebenen Fläche, zum Beispiel Tischfläche, stets flächig und damit stabil auf.

Alternativ zu dem zuvor beschriebenen Flüssigkeitskühler kann der Kühler auch durch ein Regeneratormaterial gebildet werden. Das Regeneratormaterial ist in der Lage, bei einer großen Oberfläche bzw. Wärmeaustauschfläche ohne wesentliche Strömungsverluste schnell und viel Wärme aufzunehmen. Typische Regeneratormaterialien sind: Metallwolle, Metallspäne, Metallgewebe, Drahtgestricke, Metallfaservliese, offenzellige Metallschäume, Schüttungen aus metallischem oder keramischem Granulat. Selbstverständlich ist die Erfindung nicht auf diese beispielhafte Auswahl beschränkt. Neben dem Kühleffekt bewirkt das Regeneratormaterial eine innige Durchmischung des durchströmenden Dampf-Luft-Gemisches bzw. Kondensationsaerosols, wodurch dessen Eigenschaften homogenisiert werden, zum Beispiel Konzentrationsspitzen abgebaut werden.

Der Kühler kann durch einen durchströmbaren und für die Partikel des gebildeten Kondensationsaerosols weitgehend durchlässigen Porenkörper gebildet werden.

Die gebildeten Aerosolpartikel haben einen massemedianen aerodynamischen Durchmesser (MMAD) kleiner als 2µm. Die Partikel sollen den Porenkörper möglichst ohne Verluste durchströmen. Eine Filtration der Partikel ist unerwünscht. Im Porenkörper abgeschiedene Partikel würden dessen Poren nach und nach verstopfen, was einen sprunghaften Anstieg des Druckverlustes im Porenkörper zur Folge hätte. Ferner enthalten die abgeschiedenen Partikel stets auch Nikotin. Bereits im Porenkörper abgeschiedenes Nikotin würde den Wirkungsgrad der Nikotinverabreichung schmälern. Der Porenkörper besteht typischerweise aus einem weitporigen Material, beispielsweise aus einem offenzelligen Schaummaterial, aus einem grobporigen, porösen Füllmaterial oder aus einem vliesartigen Fasermaterial. Um dennoch eine für die Kühlung und Kondensation ausreichend große Wärmeaustauschfläche zu gewährleisten, ist das Volumen des Porenkörpers ausreichend groß zu bemessen. Wie das Regeneratormaterial wirkt auch der Porenkörper auf das ihn durchströmende Dampf-Luft-Gemisch bzw. Kondensationsaerosol homogenisierend. Das verwendete Material muß außerdem gegenüber dem durchströmenden Dampf-Luft-Gemisch bzw. Kondensationsaerosol weitestgehend chemisch inert sein.

In einer bevorzugten Ausgestaltung beinhaltet der Kühler Tabak oder/und Aromastoffe. Der Tabak bzw. die Aromastoffe reichern das durchströmende Dampf-Luft-Gemisch bzw. Kondensationsaerosol mit Riech- und Geschmacksstoffen an, wodurch es vom Benutzer angenehmer zu konsumieren ist. Als Tabak eignen sich insbesondere getrockneter fermentierter Tabak, rekonstituierter Tabak, expandierter Tabak oder Mischungen derselben. Der Tabak kann beispielsweise als Schnitttabak oder gemahlener Tabak vorliegen. Als Aromastoffe eignen sich beispielhaft Tabakextrakt, Tabakaromaöle, Menthol, Kaffeeextrakt, Tabakrauch-Kondensat oder eine flüchtige aromatische Fraktion eines Tabakrauch-Kondensats.

In einer besonders bevorzugten Ausgestaltung wird der Kühler durch eine Tabakfüllung gebildet. Die Tabakfüllung vermag es, alle bisher genannten Wirkungen des Kühlers zu vereinen: Kühlung und Kondensation, Homogenisierung und Aromatisierung des durchströmenden Dampf-Luft-Gemisches bzw. Kondensationsaerosols. Bei labormäßigen Versuchen mit Prototypen wurden darüberhinaus noch weitere günstige Effekte festgestellt: beispielsweise konnte die Inhalierbarkeit des nikotinhaltigen Dampf-Luft-Gemisches und Kondensationsaerosols verbessert werden, was zum Teil gewiß auf die oben beschriebenen Effekte zurückzuführen ist. Es besteht jedoch die Hypothese, daß zusätzliche Wirkmechanismen beteiligt sind - insbesondere Diffusions- und Adsorptionsprozesse das freie, unprotonisierte Nikotin betreffend, welche im Detail noch zu erforschen wären. Die Fülldichte der Tabakfüllung ist nach oben hin dadurch beschränkt, daß die Füllung einerseits für die durchströmenden Aerosolpartikel möglichst durchlässig sein muß, und andererseits der induzierte Strömungswiderstand nicht größer sein sollte als jener von Zigaretten. Die Tabakfüllung kann aus Schnitttabak, Feinschnitt-Tabak, Stopftabak, aus einem zigarrenartigen Tabakwickel oder aus vergleichbaren oder ähnlichen Tabakformen gebildet werden. Als Tabak eignen sich insbesondere getrockneter fermentierter Tabak, rekonstituierter Tabak, expandierter Tabak oder Mischungen derselben. Der Tabak kann zusätzlich gesoßt, gewürzt, aromatisiert oder/und parfümiert werden, indem ihm aromatische Zusätze wie beispielsweise Tabakextrakt, Tabakaromaöle, Menthol, Kaffeeextrakt, Tabakrauch-Kondensat oder eine flüchtige aromatische Fraktion eines Tabakrauch-Kondensats zugesetzt werden. Die Verwendung einer Tabakfüllung als Kühler kann außerdem den Umstieg von Tabakwaren auf die erfindungsgemäße Inhalatorkomponente attraktiver machen oder/und erleichtern. In einer bevorzugten Weiterbildung der Erfindung ist vorgesehen, daß das Volumen der Tabakfüllung größer als 3cm3 ist. In eigenen labormäßigen Versuchen hat sich gezeigt, daß die oben genannten Wirkungen der Tabakfüllung erst ab dem zuvor spezifizierten Mindestvolumen in einem für den Benutzer zufriedenstellenden Maß zum Tragen kommen.

Des Weiteren ist es möglich, daß der Kühler ein Trocknungsmittel oder/und eine Aktivkohle enthält. Das Trocknungsmittel und die Aktivkohle bewirken durch ihre Adsorptionsfähigkeit und Mikroporosität eine noch weitergehende Absenkung des Ethanol- oder/und Wassergehaltes in der sie umgebenden Gasphase, und zwar sowohl während der Durchströmung des Kühlers als auch während Zeiten des Nichtgebrauches der Inhalatorkomponente.

In einer günstigen Ausgestaltungsvariante wird der Kühler durch ein Füll- oder Schüttmaterial gebildet, und das Füll- oder Schüttmaterial liegt als vorfabrizierte Packung vor. Die Verwendung einer vorfabrizierten Packung erleichtert das Assembling der Inhalatorkomponente erheblich. Die Packung braucht lediglich in das Gehäuse der Inhalatorkomponente eingesetzt zu werden. Ein aufwendiger Befüllungsprozess kann auf diese Weise vermieden werden. Die Packung kann beispielsweise aus einem Strang, insbesondere aus einem Endlosstrang, bestehend aus dem porösen Füll- oder Schüttmaterial und eine das Füll- oder Schüttmaterial umgebende Ummantelung gewonnen werden. Die Ummantelung kann beispielsweise aus Papier oder Kunststoff bestehen. Um ein Herausfallen des Füll- bzw. Schüttmaterials an den offenen Stirnseiten der Packung zu vermeiden, können die beiden Stirnseiten optional mittels einer perforierten und für das Dampf-Luft-Gemisch bzw. Kondensationsaerosol durchlässigen Abdeckung verschlossen werden. Auf solche Weise hergestellte Packungen weisen außerdem eine vergleichsweise homogene und konstant reproduzierbare Fülldichte auf.

In einer Weiterbildung ist der Kühler mehrstufig aufgebaut. Durch den mehrstufigen Kühleraufbau kann auf die Eigenschaften des Dampf-Luft-Gemisches bzw. Kondensationsaerosols noch gezielter Einfluß genommen werden, und auf diese Weise die Qualität desselben weiter verbessert werden. Den einzelnen Kühlerstufen können verschiedene Kühlerarten und -materialien oder auch nur unterschiedliche physikalische Eigenschaften zugeordnet sein.

In einer optionalen Ausgestaltung ist vorgesehen, daß der Kühler mit dem Gehäuse trennbar verbunden ist. Diese Anordnung ermöglicht es, den Kühler unabhängig von den übrigen Bestandteilen der Inhalatorkomponente auszuwechseln. Läßt die Qualität des erzeugten Dampf-Luft-Gemisches bzw. Kondensationsaerosols nach, oder ist der Kühler bereits stark mit Lösungsmittelkondensat beladen, braucht nur der Kühler ausgetauscht zu werden, während die übrigen Bestandteile der Inhalatorkomponente bis zum Erreichen ihrer vorgesehenen Lebensdauergrenze weiterverwendet werden können. Auf diese Weise kann der Gebrauch der erfindungsgemäßen Inhalatorkomponente besonders wirtschaftlich gestaltet werden.

In einer anderen optionalen Ausgestaltung umfaßt die Inhalatorkomponente ein Mundstück, über welches ein Benutzer das nikotinhaltige Dampf-Luft-Gemisch oder/und Kondensationsaerosol dargeboten erhält, und es ist vorgesehen, daß der Kühler mit dem Mundstück eine bauliche Einheit bildet, und die bauliche Einheit mit dem Gehäuse trennbar verbunden ist. Auch in dieser Ausgestaltungsvariante kann der Kühler ausgetauscht werden. Dadurch, daß der Kühler mit dem Mundstück eine bauliche Einheit bildet, gestaltet sich die Manipulation des Austausches besonders einfach, indem der Benutzer die bauliche Einheit am Mundstück ergreift und vom Gehäuse entkoppelt. Als günstiger Nebeneffekt ist zu werten, daß der Benutzer faktisch dazu gezwungen wird, das Mundstück regelmäßig durch ein neues Mundstück zu ersetzen, wenn er die Wirkungen des Kühlers aufrechterhalten möchte. Der regelmäßige, zum Beispiel tägliche Austausch des Mundstückes ist aus hygienischer Sicht zweifelsohne vorteilhaft.

Zweckmäßige und vorteilhafte Ausführungsbeispiele der Erfindung sind in den Zeichnungen dargestellt und werden in der nachfolgenden Beschreibung näher erläutert.

Es zeigen:
Fig.1 einen erfindungsgemäßen Zug-Inhalator in verschiedenen Ansichten;
Fig. 2 einen Inhalator nach Fig. 1 mit einem wiederverwendbaren Inhalatorteil und einer auswechselbaren Inhalatorkomponente im entkoppelten Zustand;
Fig. 3 das wiederverwendbare Inhalatorteil in verschiedenen Ansichten;
Fig. 4 und Fig. 5 das wiederverwendbare Inhalatorteil ohne Batteriedeckel und ohne Schaltkreisdeckel in verschiedenen Ansichten;
Fig. 6 die auswechselbare Inhalatorkomponente in verschiedenen Ansichten;
Fig. 7 die auswechselbare Inhalatorkomponente mit getrennt dargestelltem Flüssigkeitsbehälter und Mundstück;
Fig. 8 den Inhalator nach Fig. 1 ohne Schaltkreisdeckel;
Fig. 9 einen Längsschnitt durch den Inhalator nach Fig. 8 in Höhe des flächigen Verdampfers, wobei die Schnittführung abseits des Verdampfers zweckmäßig angepaßt wurde;
Fig. 10 eine Schnittansicht des Inhalators längs der Linie A-A in Fig. 9 mit Schaltkreisdeckel;
Fig. 11 einen Querschnitt des Inhalators nach Fig. 1 in Höhe des flächigen Verdampfers;
Fig. 12 das Detail a aus Fig. 10 in einer vergrößerten Darstellung;
Fig. 12a das Detail b aus Fig. 12 in einer vergrößerten Darstellung;
Fig. 13 eine alternative Ausführungsvarianten betreffend das Detail a;
Fig. 14a, Fig. 14b sowie Fig. 15a, Fig. 15b Querschnitte des flächigen Verdampfers in verschiedenen Ausführungsformen in vergrößerter Darstellung;
Fig. 16 das Detail c aus Fig. 11 in einer vergrößerten Darstellung;
Fig. 17 das Detail d aus Fig. 9 in einer vergrößerten Darstellung;
Fig. 18 eine Schnittansicht des Inhalators längs der Linie B-B in Fig. 9 mit Schaltkreisdeckel;
Fig. 19 eine Schnittansicht der auswechselbaren Inhalatorkomponente längs der Linie C-C in Fig. 7 und Fig. 11 mit angedeutetem Flüssigkeitsbehälter;
Fig. 20 eine auswechselbare Inhalatorkomponente in einer alternativen Ausführungsform in verschiedenen Schnittansichten;
Fig. 21 eine auswechselbare Inhalatorkomponente in einer weiteren alternativen Ausführungsform in einer Schnittansicht.

Fig. 1 zeigt einen erfindungsgemäßen Zug-Inhalator, dessen Form und Größe derart gestaltet sind, daß der Inhalator vom Benutzer einfach und bequem gehandhabt werden kann. Volumenmäßig ist der Inhalator nur etwa halb so groß wie eine Zigarettenschachtel. Der beispielhaft dargestellte Inhalator besteht grundsätzlich aus zwei Teilen, nämlich aus einem Inhalatorteil 1 und einer Inhalatorkomponente 2. Die Inhalatorkomponente 2 besteht aus einem Gehäuse 3 und umfaßt unter anderem einen Flüssigkeitsbehälter 4 und ein tabakpfeifenartiges Mundstück 5. Der Flüssigkeitsbehälter 4 beinhaltet Nikotin in Form einer hochverdünnten ethanolischen oder/und wässrigen Lösung, welche in der Inhalatorkomponente 2 verdampft und in ein inhalierbares Dampf-Luft-Gemisch bzw. Kondensationsaerosol übergeführt wird. Das gebildete Dampf-Luft-Gemisch bzw. Kondensationsaerosol wird dem Benutzer über das Mundstück 5 dargeboten. Der Massenanteil an Ethanol oder/und Wasser in der Nikotinlösung beträgt zusammen mindestens 50%. Um die eingangs beschriebenen vorteilhaften Wirkungen des hohen Lösungsmittelanteils möglichst weitgehend nutzbar zu machen, wird es in vielen Fällen zweckmäßig sein, den Lösungsmittelanteil deutlich über 50% zu steigern. Tabelle 1 zeigt exemplarisch die Zusammensetzung einer typischen Nikotinlösung.

**Tabelle 1:**

| Stoff | CAS-Nummer | Massen-% |
|---|---|---|
| Ethanol | 64-17-5 | 68,80 |
| Wasser | 7732-18-5 | 16,50 |
| Glycerol | 56-81-5 | 9,10 |
| Nikotin | 54-11-5 | 1,80 |
| Milchsäure | 50-21-5 | 0,23 |
| Bernsteinsäure | 110-15-6 | 0,28 |
| Lävulinsäure | 123-76-2 | 0,46 |
| Benzoesäure | 65-85-0 | 0,08 |
| Phenylessigsäure | 103-82-2 | 0,08 |
| Essigsäure | 64-19-7 | 1,67 |
| Ameisensäure | 64-18-6 | 0,53 |
| Propionsäure | 79-09-4 | 0,27 |
| Solanon | 1937-54-8 | 0,05 |
| Tabakaromaöle | *) | 0,15 |
| Ambroxid | 6790-58-5 | optional |
| Menthol | 2216-51-5 | optional |
| | Summe: | 100,00 |

| | | |
|---|---|---|
| *) mittels überkritischer CO2-Extraktion gewonnene Tabakaromaöle; z.B. Tabakextrakte der Firma Pro-Chem Specialty Limited, Hong Kong, www.pro-chem-specialty.com, z.B. Produkt-Nr. SF8010, SF8011 oder SF208118; oder gemäß Patent-Publikations-Nr. DE19654945A1, DE19630619A1, DE3218760A1, DE3148335A1 (Adam Müller et al.) hergestellte Tabakaromaöle; Voraussetzung für die Verwendung solcher Tabakaromaöle in der Nikotinlösung ist, daß diese möglichst frei von tabakspezifischen Nitrosaminen (TSNA) sind. | | |

Die exemplarisch dargestellte Nikotinlösung stellt den vorläufigen Endpunkt von eigenen umfangreichen Entwicklungsarbeiten und in vivo- Testreihen dar. Der Lösungsmittelanteil, das heißt der Anteil an Ethanol und Wasser beträgt im konkreten Beispiel 85,3 Massen-%. Das enthaltene Glycerol unterstützt durch seinen äußerst niedrigen Dampfdruck (< 0,1 Pa bei 20°C) die Aerosolbildung und hat außerdem auf Nikotin eine maskierende Wirkung, wodurch dessen organoleptischen Effekte gedämpft werden. Salopp formuliert tritt das Glycerol an die Stelle des Teers im Zigarettenrauch. Der Mix an Carbonsäuren dient zur Regulierung des pH-Wertes der Nikotinlösung und ist so abgestimmt, daß der pH-Wert des erzeugten Dampf-Luft-Gemisches bzw. Kondensationsaerosols mit den Werten von Zigarettenrauch etwa vergleichbar ist. Der pH-Wert hat einen wesentlichen Einfluß auf die Pharmakokinetik der Nikotinverabreichung. Die relativ komplexe Zusammensetzung der Carbonsäuren bewirkt außerdem ein mehrdimensionales Geschmacksempfinden, welches jenem von Zigarettenrauch ähnelt. Angemerkt sei, daß sämtliche in Tabelle 1 angeführten Carbonsäuren auch in Zigarettenrauch enthalten sind. Die exemplarisch angeführte Nikotinlösung enthält schließlich noch Solanon und mittels überkritischer CO2-Extraktion gewonnene Tabakaromaöle. Die Zugabe dieser Stoffe verleiht dem gebildeten Dampf-Luft-Gemisch bzw. Kondensationsaerosol eine typische Tabaknote. Optional können der Nikotinlösung noch weitere Aromastoffe wie Ambroxid oder Menthol zugegeben werden. Selbstverständlich ist die Erfindung nicht auf die in Tabelle 1 beispielhaft angeführte Nikotinlösung beschränkt; die Tabelle 1 soll lediglich dazu beitragen, das Verständnis der Erfindung zu vertiefen, und einen Fachmann dabei unterstützen, die Erfindung auszuführen.

Die durch Kondensation erzeugten nikotinhaltigen Aerosolpartikel weisen in der Regel einen massemedianen aerodynamischen Durchmesser (MMAD) kleiner als 2µm auf und erreichen dadurch auch die Alveolen, wo das Nikotin blitzartig in den Blutkreislauf übergeht. Schon etwa 7-10 Sekunden nach der Inhalation erreicht das Nikotin in gebündelter Konzentration sein Zielorgan - nämlich das zentrale Nervensystem.

Das Inhalatorteil 1 beinhaltet, wie nachfolgend noch im Detail erläutert wird, zumindest einen Energiespeicher und einen elektrischen Schaltkreis, wobei der Energiepeicher durch einen Batteriedeckel 6 und der Schaltkreis durch einen Schaltkreisdeckel 7 geschützt sind.

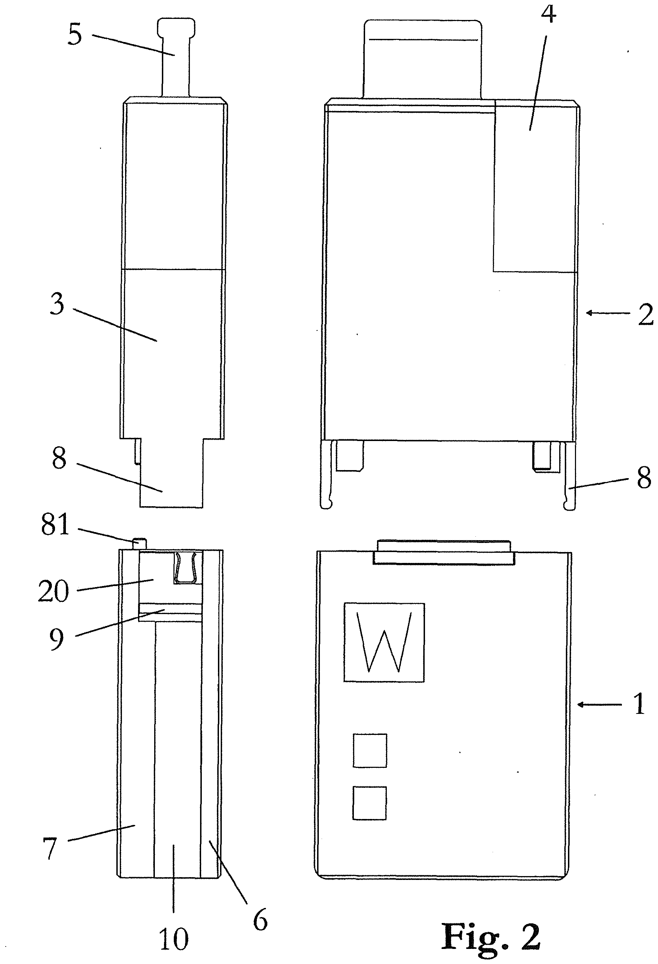

Wie die Fig. 2 zeigt, sind das Inhalatorteil 1 und die Inhalatorkomponente 2 im konkreten Ausführungsbeispiel voneinander lösbar ausgeführt. Die lösbare Kopplung besteht aus einer Schnappverbindung, gebildet aus zwei Schnapphaken 8 und zwei mit diesen zusammenwirkenden Rastnasen 9. Diese Anordnung macht das Inhalatorteil 1 wiederverwendbar, was grundsätzlich sinnvoll ist, wenn man in Betracht zieht, daß erstens das Inhalatorteil 1 mit der Nikotinlösung nicht in Berührung kommt, also nicht mit der Nikotinlösung kontaminiert wird, und zweitens Komponenten beinhaltet, welche langlebiger sind als die Bestandteile der Inhalatorkomponente 2. Die Inhalatorkomponente 2 wird, nachdem die Nikotinlösung im Flüssigkeitsbehälter 4 aufgebraucht ist, vom Benutzer als Ganzes sachgerecht entsorgt, und durch eine neue Inhalatorkomponente 2 ersetzt. Die Inhalatorkomponente 2 stellt insofern einen auswechselbaren Einwegartikel dar. Eine sachgerechte Entsorgung ist angezeigt, weil sich im Inneren des Gehäuses 3 der Inhalatorkomponente 2 im Zuge der Bildung des Dampf-Luft-Gemisches oder/und Kondensationsaerosols stets nikotinhältige Kondensatrückstände bilden und ablagern. Auch im Flüssigkeitsbehälter 4 bleiben stets Reste der Nikotinlösung zurück. Grundsätzlich wäre es natürlich auch denkbar, das Inhalatorteil 1 und die Inhalatorkomponente 2 einteilig, also voneinander untrennbar auszuführen. Diese Ausführungsform dürfte jedoch unwirtschaftlicher sein, weil in diesem Fall alle Teile und Komponenten des Inhalators, also der Inhalator als Ganzes einen Einwegartikel zur einmaligen Benutzung bildet. Selbstverständlich schließt die gegenständliche Erfindung auch diese Ausführungsform mit ein, wobei in diesem Fall der ganze Inhalator als Inhalatorkomponente aufzufassen ist.

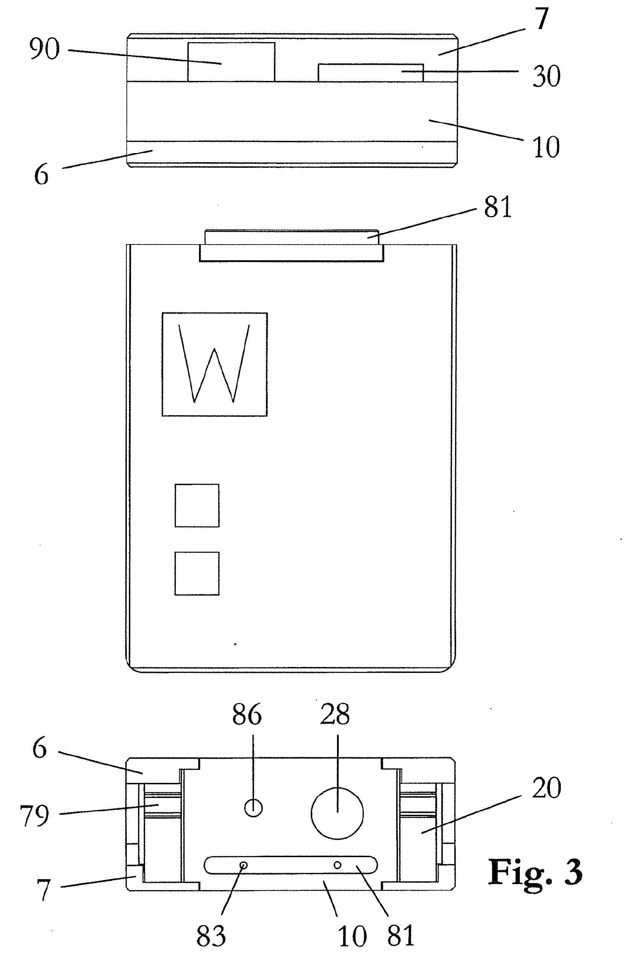

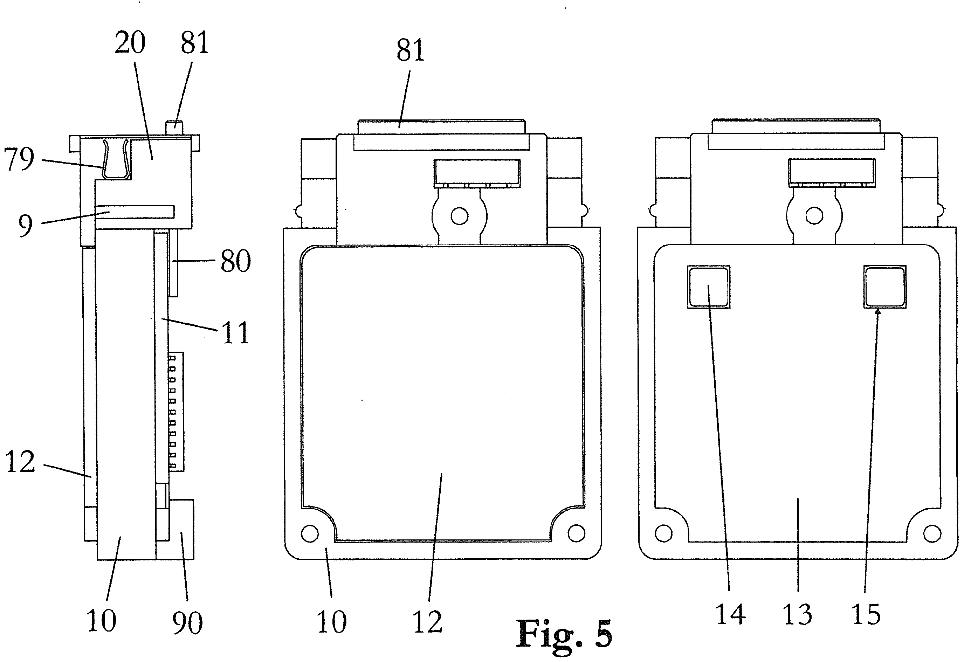

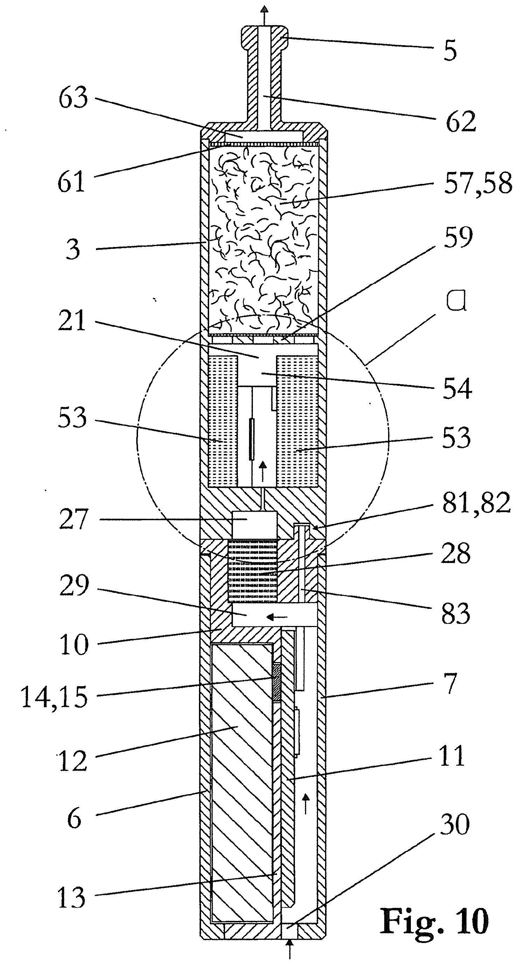

Die Fig. 3 bis 5 zeigen verschiedene Ansichten des wiederverwendbaren Inhalatorteils 1 mit und ohne Deckel. Das wiederverwendbare Inhalatorteil 1 setzt sich im Wesentlichen aus folgenden drei Gehäuseteilen zusammen: dem Batteriedeckel 6, dem Schaltkreisdeckel 7 und einem dazwischen angeordneten Trägergehäuse 10. Die drei Gehäuseteile sind aus Gewichtsgründen vorzugsweise aus Kunststoff gefertigt. Das Trägergehäuse 10 beherbergt den elektrischen Schaltkreis 11 und den Energiespeicher 12 und umfaßt eine Trennwand 13, welche den Schaltkreis 11 und den Energiespeicher 12 voneinander trennt. Der elektrische Schaltkreis 11 ist im Ausführungsbeispiel als einseitig bestückte Leiterplatte ausgebildet, welche auf der Trennwand 13 beispielsweise durch eine Klebeverbindung befestigt ist. Der Energiespeicher 12 besteht vorzugsweise aus einer wiederaufladbaren Batterie, beispielsweise aus einem Lithium-Ionen-Akkumulator oder einem Lithium-Polymer-Akkumulator, vorzugsweise in Flachbauweise. Diese Akku-Typen stellen gegenwärtig die höchsten Energiedichten und -ströme zur Verfügung und werden seit längerem vielfältig eingesetzt, wobei an erster Stelle die breite Verwendung in Mobiltelefonen zu nennen ist. Die Stromzufuhr von der Batterie 12 zur Platine 11 erfolgt über zwei Flachkontakte 14, welche auf der Rückseite der Platine 11 aufgelötet sind - siehe auch Fig. 10. Die Flachkontakte 14 ragen durch zwei etwas größere Fenster 15 in der Trennwand 13 hindurch. Die Batterie 12 umfaßt zwei korrespondierende Kontake (nicht dargestellt), welche gegen die Flachkontakte 14 gedrückt werden, wodurch ein lösbarer elektrischer Kontakt hergestellt wird. Die für diesen Zweck erforderliche Druckkraft wird vorzugsweise durch eine zwischen der Batterie 12 und dem Batteriedeckel 6 angeordnete Blattfeder (nicht dargestellt) erzeugt. Der Batteriedeckel 6 ist mit dem Trägergehäuse 10 lösbar verbunden - im Ausführungsbeispiel mittels einer Schraubverbindung (siehe Fig. 1). Selbstverständlich könnte der Batteriedeckel 6 alternativ auch als ein verrastbarer Schiebedeckel ausgebildet sein. Der Schaltkreisdeckel 7 ist mit dem Trägergehäuse 10 vorzugsweise untrennbar verbunden, zum Beispiel mittels einer Klebe- oder Schweißverbindung. Auf diese Weise soll einer unbefugten Manipulation des Schaltkreises 11 entgegengewirkt werden. Im normalerweise seltenen Fall eines Schaltkreisdefektes ist das ganze Inhalatorteil 1 mit Ausnahme der Batterie 12 zu ersetzen. Weitere Bestandteile und Eigenschaften des wiederverwendbaren Inhalatorteils 1 werden später noch näher beschrieben.

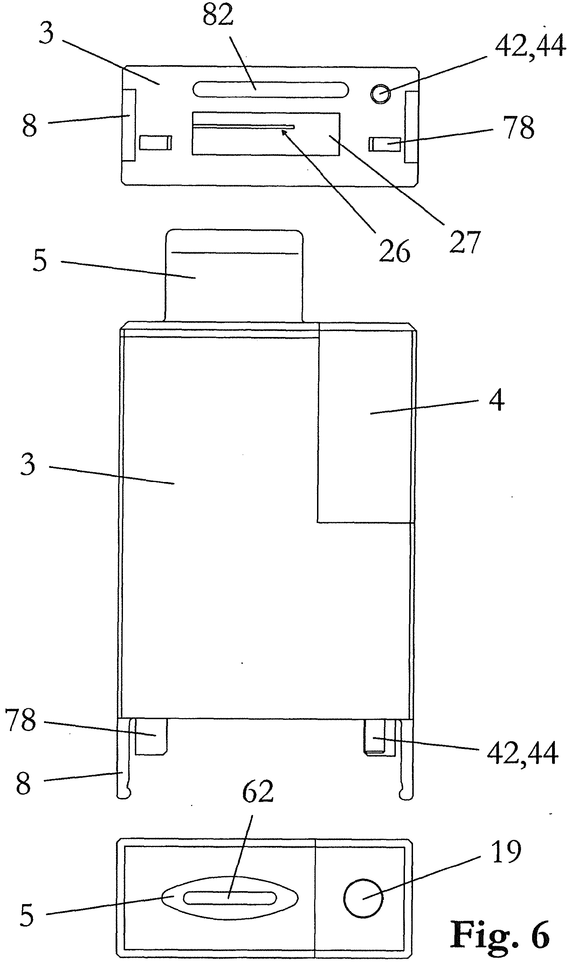

Die Fig. 6 und 7 zeigen verschiedene Ansichten der auswechselbaren Inhalatorkomponente 2. Wie schon erwähnt wurde, wird die auswechselbare Inhalatorkomponente 2 im Wesentlichen vom Gehäuse 3 ausgebildet, und beinhaltet unter anderem den Flüssigkeitsbehälter 4 und das tabakpfeifenartige Mundstück 5. Der Flüssigkeitsbehälter 4 und das Mundstück 5 sind untrennbar mit dem Gehäuse 3 verbunden. Herstellungstechnisch ist es günstig, den Flüssigkeitsbehälter 4 und das Mundstück 5 als separate Teile zu fertigen, und erst in einem Folgeschritt mit dem Gehäuse 3 beispielsweise durch eine Klebe- oder Schweißverbindung zu verbinden - siehe Fig. 7. Grundsätzlich ist es natürlich auch denkbar, den Flüssigkeitsbehälter 4 oder/und das Mundstück 5 mit dem Gehäuse 3 einteilig auszubilden. Das Gehäuse 3, der Flüssigkeitsbehälter 4 und das Mundstück 5 sind aus Gewichtsgründen vorzugsweise aus Kunststoff gefertigt, wobei bei der Materialauswahl für den Flüssigkeitsbehälters 4 Kunststoffe gemäß US 5,167,242 (James E. Turner et al.) und US 6,790,496 (Gustaf Levander et al.) Verwendung finden können.

Die Befüllung des Flüssigkeitsbehälters 4 mit der Nikotinlösung 16 erfolgt über ein Füllloch 17 vorzugsweise unter Schutzgasatmosphäre wie Argon oder Stickstoff. An einer Stirnseite des Flüssigkeitsbehälters 4 befindet sich ein klappenartiger, öffenbarer Verschluß 18, welcher vom Benutzer vor der Verwendung der Inhalatorkomponente 2 durch Eindrücken geöffnet wird. Der öffenbare Verschluß 18 wird später noch im Detail beschrieben. Der Flüssigkeitsbehälter 4 wird niemals vollständig mit der Nikotinlösung 16 gefüllt. Eine vollständige Befüllung würde wegen der Inkompressibilität der Nikotinlösung 16 dazu führen, daß sich der klappenartige, öffenbare Verschluß 18, welcher stets eine gewisse Elastizität aufweist, nicht mehr eindrücken und öffnen ließe. Nach der Befüllung wird das Füllloch 17 mit einem Verschlußdeckel 19 luftdicht verschlossen. Der Verschlußdeckel 19 kann beispielsweise aufgeklebt oder aufgeschweißt werden, wobei eine Hitzeeinwirkung auf die Nikotinlösung 16 möglichst vermieden werden sollte. Alternativ kann das Füllloch 17 als Kapillarbohrung ausgebildet sein, und die Befüllung mit der Nikotinlösung 16 über eine Injektionsnadel erfolgen. In diesem Fall könnte der Verschlußdeckel 19 entfallen, und die Kapillarbohrung selbst zugeschmolzen werden. Weitere Bestandteile und Eigenschaften der auswechselbaren Inhalatorkomponente 2 werden später noch im Detail beschrieben.

Fig. 8 zeigt den Inhalator nach Fig. 1 mit abgehobenem Schaltkreisdeckel 7. Unter anderem zeigt die Fig. 8 die Schnappverbindung, bestehend aus den zwei Schnapphaken 8 und den korrespondierenden Rastnasen 9, im gekoppelten, eingerasteten Zustand. Dabei sind die Schnapphaken 8 als Fortsätze des Gehäuses 3 ausgebildet, während die Rastnasen 9 von Kontaktelementen 20 gebildet werden. Die Kontaktelemente 20 sind am Trägergehäuse 10 des wiederverwendbaren Inhalatorteils 1 durch eine Klebeverbindung befestigt und erfüllen noch weitere Funktionen, welche später noch im Detail beschrieben werden.

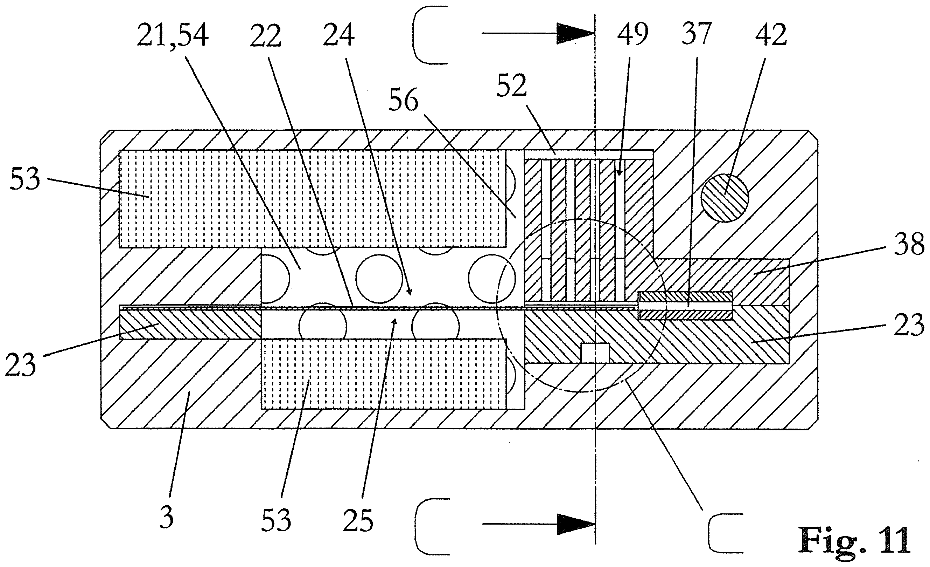

Die Fig. 9 bis 13 geben nähere Aufschlüsse über das Innenleben des Inhalators und über dessen grundsätzliche Funktionsweise. Demnach bildet das Gehäuse 3 der auswechselbaren Inhalatorkomponente 2 im Inneren eine Kammer 21. Die Kammer 21 wird, wie Fig. 11 am besten zeigt, von einem Verdampfer 22 brückenartig und damit berührungsfrei durchsetzt. Der Verdampfer 22 hat im Ausführungsbeispiel eine folien- bzw. streifenförmige Gestalt und besteht aus einem elektrischen Widerstandsheizelement und einem Docht, welche zusammen einen flächigen Verbund bilden. Die Kapillarstruktur des Dochts ist dazu geeignet, die flüssige Nikotinlösung 16 aufzusaugen. Das Heizelement und der Docht können auf verschiedenste Weise ausgebildet und miteinander verbunden sein. Beispielhafte Ausbildungsformen werden später noch beschrieben. Der flächige Verdampfer 22 lagert mit zwei Endabschnitten auf zwei elektrisch leitenden, plattenförmigen Kontakten 23, auf deren Oberfläche er auch gleichzeitig elektrisch kontaktiert ist. Die elektrische Kontaktierung des Verdampfers 22 auf den plattenförmigen Kontakten 23 kann durch eine Schweißverbindung erfolgen. Die Schweißverbindung kann durch Punktschweißen, Widerstandsschweißen, Ultraschallschweißen, Laserschweißen, Bonden oder sonstige geeignete Schweißverfahren hergestellt werden. Hierbei ist es vorteilhaft, wenn die plattenförmigen Kontakte 23 aus demselben oder aus einem ähnlichen Material bestehen wie das Heizelement, woraus sich günstige Voraussetzungen für eine Schweißverbindung als Kontaktierungsverfahren ergeben. Es ist darauf zu achten, daß der Docht bzw. dessen Kapillarstruktur durch die Schweißung nach Möglichkeit nicht beeinträchtigt wird. Bei Bedarf ist die Schweißung nur punktuell auszuführen. Alternativ erfolgt die elektrische Kontaktierung des flächigen Verdampfers 22 durch eine Klebeverbindung mittels eines elektrisch leitenden Klebstoffes, z.B. mittels eines silberhaltigen Klebers auf Epoxidbasis. Solche Klebstoffe können beispielsweise von der Firma Epoxy Technology, www.epotek.com bezogen werden. In diesem Fall können die plattenförmigen Kontakte 23 im Prinzip aus jedem beliebigen elektrischen Kontaktwerkstoff hergestellt werden, solange der Werkstoff mit dem verwendeten Klebstoff kompatibel ist; besonders günstig ist es, wenn die plattenförmigen Kontakte 23 durch Leiterplatten gebildet werden, welche sich durch den Vorteil eines besonders geringen Gewichts auszeichnen.

Der Bereich zwischen den beiden plattenförmigen Kontakten 23 definiert im Ausführungsbeispiel einen beheizten Abschnitt des Verdampfers 22, welcher berührungsfrei in der Kammer 21 angeordnet ist. Die berührungsfreie Anordnung führt dazu, daß die Wärmeleitungsverluste in Dickenrichtung des flächigen Verdampfers 22 gleich null sind. Dadurch kann sich dieser Abschnitt so weit aufheizen, daß die im Docht gespeicherte Nikotinlösung 16 Siedetemperatur erreicht und verdampft. Die Kapillarstruktur des Dochts liegt im besagten Abschnitt zumindest auf einer Seite des flächigen Verdampfers 22 weitgehend frei. Diese Seite ist, wie später noch im Zuge der Beschreibung beispielhafter Ausbildungsformen des Verdampfers verständlich gemacht wird, vorzugsweise die den plattenförmigen Kontakten 23 abgewandte Seite 24 des flächigen Verdampfers 22. Der im Zuge der Verdamfung der Nikotinlösung gebildete Dampf kann also weitflächig und ohne wesentliche Behinderung aus der freiliegenden Kapillarstruktur des Dochts ausströmen. In einer zweiten Ausgestaltung des Verdampfers 22, welche ebenfalls später noch anhand von Beispielen beschrieben wird, liegt die Kapillarstruktur des Dochts im besagten Abschnitt zusätzlich auf der der Seite 24 gegenüberliegenden Seite 25 des flächigen Verdampfers 22 weitgehend frei, so daß sich die Verdampfungsfläche und folglich auch die maximal erzielbare Verdampfungsleistung gegenüber dem zuerst genannten Fall verdoppelt. Die maximal erzielbare Verdampfungsleistung sei durch das erstmalige Auftreten einer Siedekrise im Docht definiert.

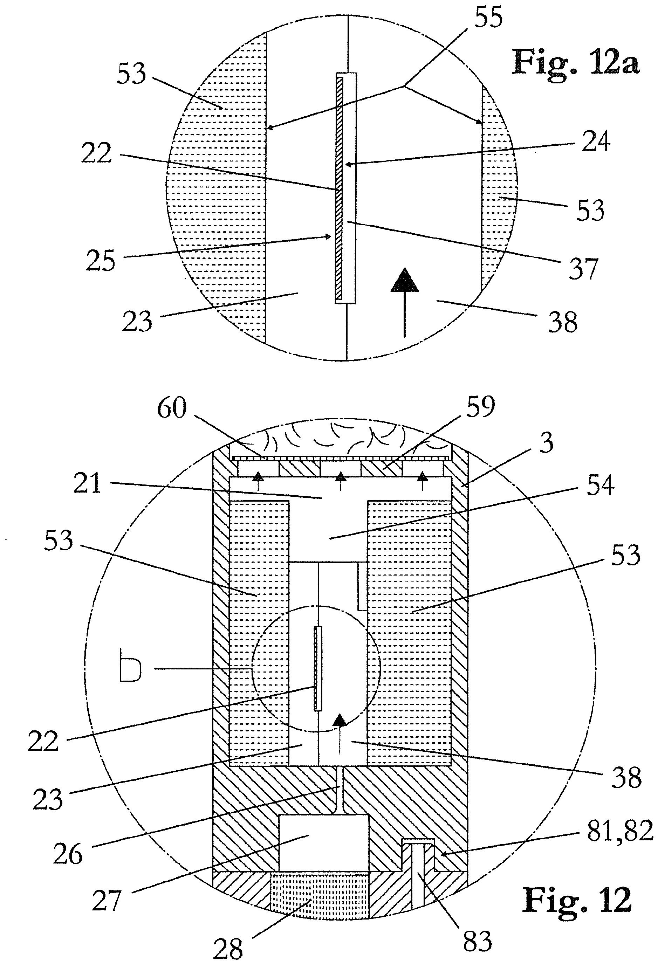

Das Gehäuse 3 bildet ferner eine Lufteinlaßöffnung 26 für die Zufuhr von Luft aus der Umgebung in die Kammer 21. Die zugeführte Luft mischt sich in der Kammer 21 mit dem aus der freiliegenden Kapillarstruktur des Dochts ausströmenden Dampf, im Zuge dessen sich das Dampf-Luft-Gemisch bzw. Kondensationsaerosol ausbildet. Die Lufteinlaßöffnung 26 ist als schlitzförmiger Kanal ausgebildet. Der schlitzförmige Kanal ist parallel zum flächigen Verdampfer 22 ausgerichtet. Im Ausführungsbeispiel nach Fig. 10 bzw. Fig. 12 ist der schlitzförmige Kanal etwas seitlich versetzt zum flächigen Verdampfer 22, nämlich auf jener Seite des flächigen Verdampfers angeordnet, auf welcher die Kapillarstruktur des Dochts weitgehend freiliegt. Durch diese Anordnung wird erreicht, daß die, durch den schlitzförmigen Kanal 26 in die Kammer 21 einströmende Luft die freiliegende Kapillarstruktur des Dochts komplett überströmt, und sich homogene Mischungsbedingungen einstellen können. Durch Variation der Schlitzhöhe des schlitzförmigen Kanals 26 kann, wenn man ein konstantes Zugprofil (Zugvolumen, Zugdauer) voraussetzt, die Strömungsgeschwindigkeit der einströmenden Luft verändert werden, und auf diese Weise auf die Dynamik der Aerosolbildung und auf die Eigenschaften des erzeugten Aerosols in gewissen Grenzen Einfluß genommen werden. Eine Verringerung der Strömungsgeschwindigkeit läßt die Aerosolteilchen im Mittel an Größe zunehmen. Auch die geometrische Lage des schlitzförmigen Kanals 26 in Bezug auf den flächigen Verdampfer 22 hat einen Einfluß auf die Aerosolbildung.

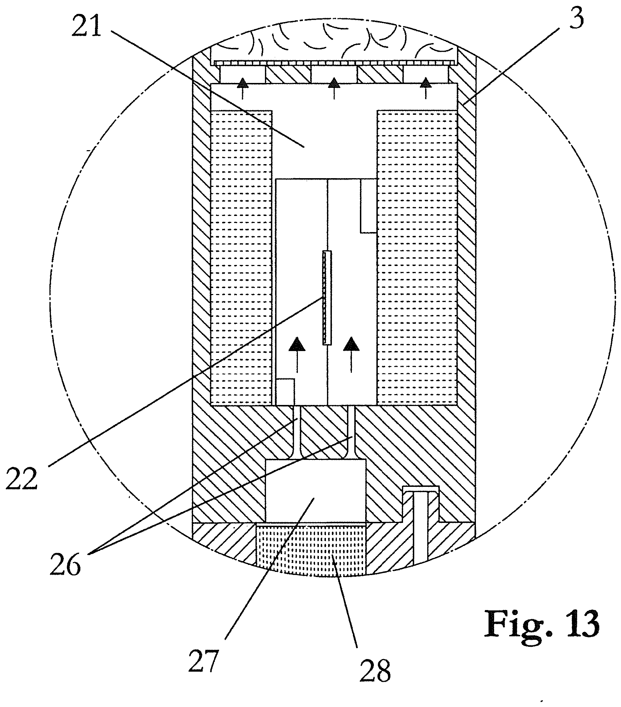

Die Fig. 13 zeigt eine alternative Anordungen der Lufteinlaßöffnung 26: demnach wird die Lufteinlaßöffnung 26 durch zwei schlitzförmige Kanäle 26 gebildet, welche auf gegenüberliegenden Seiten des flächigen Verdampfers 22 angeordnet sind. Der flächige Verdampfer 22 wird also auf beiden Seiten von der in die Kammer 21 einströmenden Luft umströmt. Diese Anordnung eignet sich sich vor allem für die Ausführungsvariante des flächigen Verdampfers 22, bei welcher die Kapillarstruktur des Dochts beidseitig freiliegt, da in diesem Fall von beiden Seiten 24 und 25 des flächigen Verdampfers 22 Dampf abströmt. Sie ist jedoch ebenso für die Ausführungsvariante des flächigen Verdampfers 22 mit nur einseitig freiliegender Kapillarstruktur geeignet, insofern als der zweite Luftstromanteil, welcher den Verdampfer dann quasi passiv umströmt, den ersten, die Aerosolbildung bewirkenden Luftstromanteil abschwächt, wodurch wiederum Einfluß auf die Eigenschaften des gebildeten Aerosols genommen werden kann.

Die als schlitzförmiger Kanal ausgebildete Lufteinlaßöffnung 26 bezieht die Luft aus einer Plenumkammer 27, welche dazu dient, die Luft gleichmäßig auf den schlitzförmigen Kanal 26 zu verteilen, sodaß im schlitzförmigen Kanal im Wesentlichen allseits gleiche Strömungsbedingungen herrschen. Stromaufwärts der Plenumkammer 27 befindet sich eine Strömungsdrossel 28. Die Strömungsdrossel 28 hat den Zweck, einen Strömungswiderstand zu erzeugen, welcher dem einer Zigarette ähnlich ist, so daß der Benutzer während eines Zuges einen ähnlichen Zugwiderstand verspürt wie bei einem Zug an einer Zigarette. Konkret sollte der Strömungswiderstand bei einem Durchfluß von 1,05 L/min im Bereich 12-16mbar liegen und eine möglichst lineare Charakteristik aufweisen. Die Strömungsdrossel 28 kann beispielsweise aus einem offenporigen Sinterkörper aus Metall oder Kunststoff gebildet sein, dessen Poren von der Luft durchströmt werden. In Prototypen haben sich beispielsweise poröse Kunststoffformkörper der Firma Porex, www.porex.com bewährt. Im Ausführungsbeispiel ist die Plenumkammer 27 Teil der auswechselbaren Inhalatorkomponente 2 und die Strömungsdrossel 28 Teil des wiederverwendbaren Inhalatorteils 1. Grundsätzlich wäre es auch möglich, die Plenumkammer 27 und die Strömungsdrossel 28 in der auswechselbaren Inhalatorkomponente 2 anzuordnen, oder alternativ beide im wiederverwendbaren Inhalatorteil 1 anzuordnen.

Die Fig. 10 zeigt den weiteren Verlauf der Luftströmung stromaufwärts der Strömungsdrossel 28. Die Strömung ist durch Pfeile angedeutet. Demnach bezieht die Strömungsdrossel 28 die Luft aus einem Querkanal 29, welcher seinerseits in den Raum zwischen der Platine 11 und dem Schaltkreisdeckel 7 mündet. Die eigentliche Zufuhr der Luft aus der Umgebung erfolgt über eine vom Schaltkreisdeckel 7 gebildete Speiseöffnung 30. Die Speiseöffnung 30 ist auf der dem Mundstück 5 gegenüberliegenden Stirnseite des Inhalators angeordnet. Diese Lage schützt am ehesten vor dem Eintritt von Regenwasser.

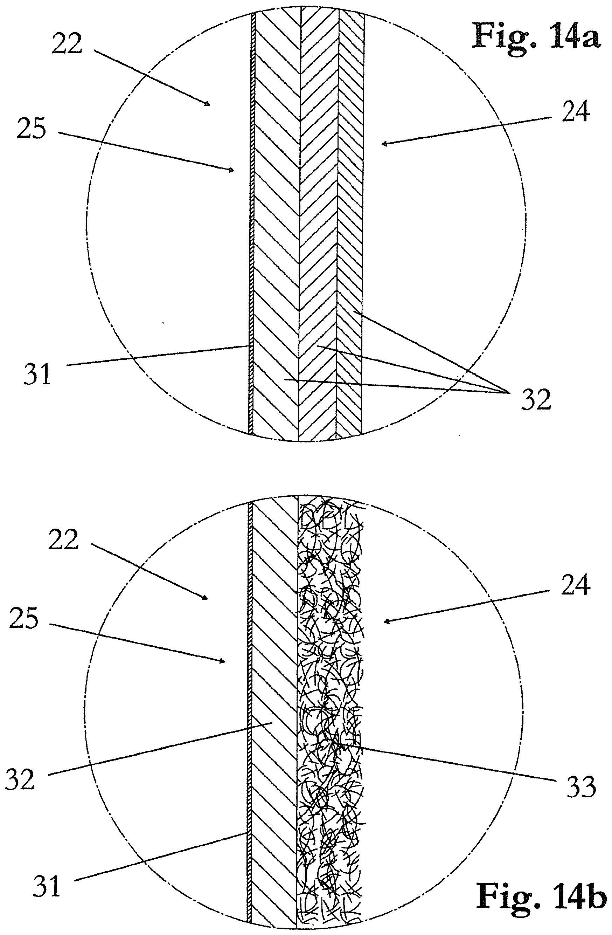

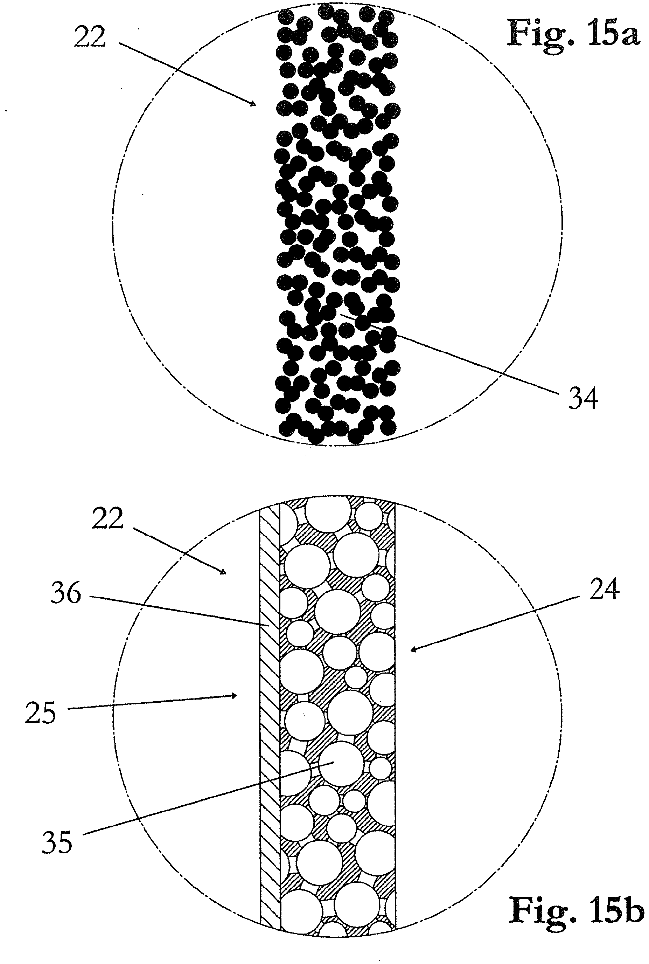

Die Fig. 14a, 14b und 15a, 15b, 15c zeigen beispielhafte Ausbildungsformen des flächigen Verdampfers 22 anhand von Querschnittsdarstellungen, wobei unter "Querschnitt" ein Schnitt normal zur Längsrichtung des flächigen Verbundes verstanden wird (vgl. Fig. 9). Konkret zeigen die Fig. 14a und 14b Ausführungsformen mit nur einseitig freiliegender Kapillarstruktur, während die Fig. 15a und 15b Ausführungsformen zeigen, bei welchen die Kapillarstruktur des Dochts auf beiden Seiten des flächigen Verdampfers freiliegt. Nach der Ausführungsform gemäß Fig. 14a besteht der Verdampfer 22 aus einem Verbund von vier Lagen: nämlich aus einer Metallfolie 31 und drei darauf aufgesinterten Metalldrahtgeweben 32. Das Metall besteht aus Edelstahl (z.B. AISI 304 oder AISI 316), oder aus einer Heizleiterlegierung - insbesondere aus der Gruppe der NiCr-Legierungen oder CrFeAI-Legierungen ("Kanthal"). Bei Verwendung von Edelstahl wird kohlenstoffreduzierten Chargen (z.B. AISI 304L oder AISI 316L) der Vorzug gegeben, weil diese weniger anfällig für interkristalline Korrosion sind. Die Metallfolie 31 kann in Edelstahlausführung beispielsweise von der Firma Record Metall-Folien GmbH, www.recordmetall.de bezogen werden. Die Drahtgewebe können beispielsweise von den Firmen Haver & Boecker, www.haverboecker.com oder Spörl KG, www.spoerl.de bezogen werden. Die vier Lagen sind durch eine Sinterung miteinander verbunden. Die Sinterung wird vorzugsweise im Vakuum oder unter Wasserstoff-Schutzgas durchgeführt. Solche Sinterungen zählen zum Stand der Technik und werden beispielsweise von der Firma GKN Sinter Metals Filters GmbH, www.gkn-filters.com sowie von der Firma Spörl KG, www.spoerl.de routinemäßig durchgeführt. Die Sinterung erfolgt vorteilhafterweise in Form eines Vielfachnutzens; das heißt, daß nicht einzelne flächige Verbunde gesintert werden, sondern größere flächige Nutzen beispielsweise im Format 200x200mm. Die einzelnen Verdampfer werden nach der Sinterung durch Laserschneiden oder Stanzen aus dem Vielfachnutzen gewonnen und anschließend optional in einem Beizbad geätzt. Tabelle 2 zeigt beispielhaft die Spezifikationen von in Prototypen eingesetzten flächigen Verbunden bzw. Verdampfern 22.

**Tabelle 2:**

| | | |
|---|---|---|
| Metallfolien-Dicke: | 10µm | |
| Metallfolien-Material: | AISI 304 | |
| 1. Drahtgewebelage: | 36×90µm | Drahtdurchmesser × Maschenweite |
| 2. Drahtgewebelage: | 30×71µm | Drahtdurchmesser × Maschenweite |
| 3. Drahtgewebelage: | 20×53µm | Drahtdurchmesser × Maschenweite |
| Drahtgewebe-Material: | AISI 316L | |
| Verbund-Spannweite: | 14mm | |
| Verbund-Breite: | 2-5mm | |
| Verbund-Dicke: | 140-160µm | |
| Ätzrate: | bis 50% | mit Badbeize Avesta 302 *) |
| Porosität: | 65-80% | abhängig von der Ätzrate |

| | | |
|---|---|---|
| *) Hersteller: Avesta Finishing Chemicals, www.avestafinishing.com | | |

Die Verbund-Spannweite entspricht jener Strecke in der Kammer 21, welche der Verdampfer 22 berührungsfrei überbrückt; im konkreten Ausführungsbeispiel entspricht diese Strecke dem Abstand zwischen den beiden plattenförmigen Kontakten 23. Die Verbund-Spannweite und die Verbund-Breite haben einen entgegengesetzten Einfluß auf den resultierenden Heizelement-Widerstand. Die Ätzrate definiert den insgesamt durch die Ätzung erzielten Massenverlust. Die erste Drahtgewebelage liegt direkt auf der Metallfolie 31 auf. Die dritte Drahtgewebelage bildet die Decklage und gleichzeitig die freiliegende Kapillarstruktur des flächigen Verdampfers 22. Der flächige Verdampfer 22 lagert vorzugsweise mit der Metallfolie 31 auf den plattenförmigen Kontakten 23. Die elektrische Kontaktierung der Metallfolie 31 erfolgt vorzugsweise über eine flächige Klebeverbindung zwischen der Metallfolie 31 und den elektrisch leitenden, plattenförmigen Kontakten 23. Grundsätzlich könnte die Kontaktierung auch durch eine Schweißverbindung hergestellt werden. Ein auf solche Weise kontaktierter flächiger Verdampfer 22 mit den Spezifikationen gemäß Tabelle 2, mit einer Verbund-Breite von 2mm und einer Ätzrate von 35% weist einen Heizelement-Widerstand von etwa 310mOhm auf. Bei Verwendung von Heizleiterlegierungen anstatt Edelstahl kann der Heizelement-Widerstand deutlich gesteigert werden, konkret bei Verwendung von DIN-Werkstoff-Nummer 2.4872 (NiCr20AISi) im Vergleich zu AISI 304/ AISI 316 um den Faktor 1,8 und bei Verwendung von DIN-Werkstoff-Nummer 1.4765 (CrAl255) gar um den Faktor 2,0. Folgedessen hätte ein flächiger Verdampfer mit einer Verbund-Breite von 5mm in Ausführung DIN-Werkstoff-Nummer 2.4872 aber ansonsten gleichen Spezifikationen, wie zuvor angeführt, einen Heizelement-Widerstand von etwa 225 mOhm. Erfolgt die Energiezufuhr auf Basis einer Lithium-Polymer-Zelle mit einer Nenn- oder Leerlaufspannung von 3,7V und einer Nutzspannung unter Last von ca. 3,1V, errechnet sich auf Basis des Ohmschen Gesetzes der Strom, welcher durch den flächigen Verdampfer fließt, zu 10A (für 310mOhm) bzw. 13,8A (für 225mOhm). Diese Stromstärken können aus heutigen Lithium-Polymer-Zellen problemlos bezogen werden. In einem weiteren Schritt errechnet sich die elektrische Nennleistung, das ist gleichzeitig die maximal realisierbare Heizleistung zu 31W (für 310mOhm) bzw. 42,7W (für 225mOhm). Wie später noch beschrieben wird, können diese Leistungen durch den elektrischen Schaltkreis 11 beliebig reduziert werden.

Auf Basis der zuvor angeführten Spezifikationen eines beispielhaften flächigen Verdampfers mit einer Verbund-Breite von 5mm und einer Ätzrate von 35% errechnet sich das Porenvolumen des flächigen Verdampfers im Abschnitt der Verbund-Spannweite (Verdampfungsabschitt) zu etwa 7,5µL. Dieses Volumen wird von der zu verdampfenden Nikotinlösung 16 ausgefüllt und entspricht jener Menge an flüssigem Material, welche pro Zug (intermittierender Inhalatorbetrieb) maximal verdampft werden kann. Legt man beispielhaft die in Tabelle 1 angeführte Nikotinkonzentration in Höhe von 1,8 Massen-% bzw. 1,5 Vol.-% zugrunde, so resultiert daraus theoretisch pro Verdampfung bzw. Zug eine maximal freigesetzte Nikotin-Dosis von 110µg bzw. aufgerechnet auf 10 Inhalationen eine Gesamtdosis von 1,1mg. Real wird die maximal erzielbare Dosis aus verschiedenen Gründen etwas unter den berechneten Werten liegen. Wesentlich ist jedoch die Tatsache, daß mit dem Inhalator die Nikotin-Dosen von heutigen Zigaretten (0,1-1,0mg) problemlos verabreicht werden können. Wesentlich ist ferner, daß sich die Wirkstoff-Dosis beliebig reduzieren läßt, sei es durch eine Verringerung der Wirkstoffkonzentration im flüssigen Material, sei es durch die Wahl einer kleineren Verbund-Breite, oder sei es durch eine Drosselung der zugeführten Heizleistung mittels des elektrischen Schaltkreises 11. Die letztere Maßnahme wirkt außerdem einer thermischen Zersetzung des flüssigen Materials 16 entgegen, da der Verbund 22 nicht so hoch aufgeheizt wird.

Es sei angemerkt, daß sowohl die Metallfolie 31 als auch die auf die Folie aufgesinterten Metalldrahtgewebe 32 einen Beitrag zum elektrischen Heizwiderstand leisten. Der elektrische Heizwiderstand kann insofern als Parallelschaltung von diesen Einzelwiderständen interpretiert werden. Ebenso ist auch die Kapillarwirkung des Dochts im Zusammenwirken der Drahtgewebe 32 mit der Metallfolie 31 begründet, wobei auch schon eine einzelne Drahtgewebelage in Kombination mit der Metallfolie 31 einen Kapillareffekt erzeugen kann.

Fig. 14b zeigt eine zweite exemplarische Ausführungsform eines flächigen Verdampfers 22 mit nur einseitig freiliegender Kapillarstruktur. Diese Ausführungsform unterscheidet sich von jener nach Fig. 14a nur dadurch, daß anstatt der äußeren zwei Drahtgewebelagen ein Faserverbund in Form eines Vlieses 33 vorgesehen ist, welches auf die erste Drahtgewebelage 32 aufgesintert ist. Solche Vliese 33 können in Edelstahlausführung beispielsweise von der Firma GKN Sinter Metals Filters GmbH, www.gkn-filters.com nach Kundenspezifikation angefertigt werden. Das Vlies 33 hat vorzugsweise eine Dicke von 100-300µm und eine Porosität >70%. Das die freiliegende Kapillarstruktur des Dochts bildende Vlies 33 weist im Vergleich zu den Drahtgeweben 32 eine deutlich größere Oberfläche auf; die größere Oberfläche wirkt sich günstig auf den Verdampfungsprozess aus. Der flächige Verbund 22 kann nach der Sinterung optional wieder geätzt werden.

Fig. 15a zeigt eine Ausführungsform eines flächigen Verdampfers 22 mit beidseitig freiliegender Kapillarstruktur. Der Verdampfer besteht demnach aus einer offenporigen Sinterstruktur gebildet aus einem homogenen körnigen, faserigen oder flockigen Sinterverbund 34. Die Herstellung von dünnen porösen Sinterverbunden ist seit langem bekannt. JP 2004/332069 (Tsujimoto Tetsushi et al., Mitsubishi Materials Corporation) beschreibt beispielsweise ein Verfahren zur Herstellung von dünnen porösen Sinterverbunden aus Metall im bevorzugten Dickenbereich von 50-300µm, welches sich dadurch auszeichnet, daß dem zu verarbeitenden Metallpulver entfernbare Füllstoffe, im konkreten Fall Acrylharz-Kügelchen beigemischt werden. Die Acrylharz-Kügelchen sind Platzhalter, welche im Zuge einer Wärmebehandlung noch vor der eigentlichen Sinterung bei etwa 500°C im Vakuum praktisch rückstandslos sublimieren und Hohlräume zurücklassen, welche Hohlräume auch während und nach der Sinterung bestehen bleiben. Auf diese Weise wurden fläche Verbunde bestehend aus Edelstahl der Spezifikation AISI 316L mit Porositäten von typischerweise 70-90% hergestellt. Das Verfahren ist geeignet, einen Verdampfer 22 zur Verwendung in einer erfindungsgemäßen Inhalatorkomponente 2 herzustellen. Zu beachten ist lediglich, daß der mittlere Porendurchmesser im homogenen Sinterverbund möglichst >10µm ist, um eine ausreichend schnelle Infiltration des Dochts mit der Nikotinlösung 16 sicherzustellen. Die Korngröße der zu verarbeitenden Metallpulver und der Acrylharz-Kügelchen sind auf diese Bedingung abzustimmen. Der im Verfahren nach JP 2004/332069 angeführte, bevorzugte Dickenbereich von 50-300µm deckt sich mit dem für den flächigen Verdampfer 22 besonders bevorzugten Dickenbereich. Zur weiteren Steigerung der Porosität und des elektrischen Widerstandes kann der Sinterverbund 34 nach der Sinterung optional wieder geätzt werden. Die Befestigung und Kontaktierung des flächigen Sinterverbundes 34 auf den plattenförmigen Kontakten 23 erfolgt vorzugsweise durch eine Schweißverbindung. Eine Klebeverbindung ist nur möglich, wenn der verwendete Klebstoff eine hinreichend pastöse oder zähflüssige Konsistenz aufweist. Andernfalls bestünde die Gefahr, daß der Klebstoff in die Porenstruktur des Verbundes eintritt und die Kapillarwirkung des Dochts beeinträchtigt.

Fig. 15b zeigt schließlich eine weitere Ausführungsform eines Verdampfers 22 mit beidseitig freiliegender Kapillarstruktur. Demnach besteht der flächige Verdampfer aus einem offenporigen Schaum 35 gebildet aus einem elektrischen Widerstandsmaterial. Ein solches Schaummaterial kann in Edelstahlausführung (z.B. AISI 316L) von der Firma Mitsubishi Materials Corporation, www.mmc.co.jp bezogen werden. Hierbei wird von einem standardmäßigen Schaummaterial mit einer Dicke von 0,5mm, einem Porendurchmesser im Bereich 50-150µm und einer Porosität von zirka 90% ausgegangen, welches Material durch Walzen in der Dicke beliebig bis auf etwa 100µm verdichtet werden kann. Das verdichtete Material kann anschließend optional noch gesintert werden. Durch die Verdichtung sinkt natürlich auch die Porosität, welche aber bei Bedarf im Zuge einer abschließenden Ätzbehandlung wieder gesteigert werden kann. Der flächige Verdampfer 22 kann aus einer einzigen Schaumlage oder aus mehreren, miteinander versinterten Schaumlagen bestehen. Zur Steigerung der Stabilität und Festigkeit des flächigen Verbundes 22 kann der Schaum 35 optional auf eine dünne Trägerlage 36, beispielsweise auf ein Drahtgewebe aus Edelstahl aufgesintert werden. Bezüglich der Befestigung und Kontaktierung des Schaums 35 auf den plattenförmigen Kontakten 23 gilt Gleiches, wie schon in Zusammenhang mit der Ausführungsform gemäß Fig. 15a ausgeführt wurde.

Angemerkt werden soll, daß bei den Ausführungsformen nach Fig. 15a und 15b anstatt Edelstahl selbstverständlich auch Heizleiterlegierungen, insbesondere aus der Gruppe der NiCr-Legierungen und CrFeAI-Legierungen ("Kanthal") verfahrensgemäß verarbeitet werden können.

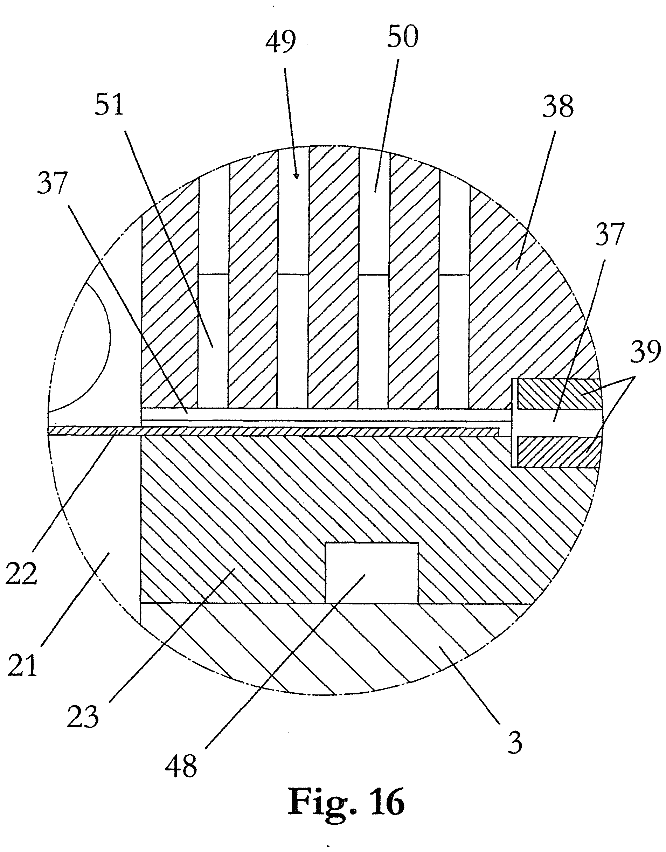

Im Folgenden soll die Versorgung des Verdampfers 22 mit der Nikotinlösung 16 näher beschrieben werden. Wie Fig. 12a und Fig. 16 zeigen, ragt der Verdampfer 22 mit einem Ende in einen Kapillarspalt 37. Der Kapillarspalt 37 speist den Docht des Verdampfers mit der flüssigen Nikotinlösung 16; wie den Figuren entnommen werden kann, ist der Querschnitt des Kapillarspalts 37 größer als der Querschnitt des Verdampfers 22. Dies hat den Effekt, daß die Nikotinlösung 16 hauptsächlich durch den lichten Querschnitt des Kapillarspalts 37 zur Verdampfungszone strömt, wodurch der Docht schneller infiltriert werden kann, und die Wartezeit zwischen zwei Zügen bzw. Inhalationen verkürzt werden kann. Der Effekt wirkt zumindest bis zur Mündung des Kapillarspalts 37 in die Kammer 21. Ab diesem Punkt ist allein der Docht des Verdampfers 22 für den Flüssigkeitstransport verantwortlich. Der Kapillarspalt 37 wird im Grunde durch einen der beiden plattenförmigen Kontakte 23 und ein auf diesen flächig aufgesetztes Oberteil 38 gebildet, indem in das Oberteil 38 und in den plattenförmigen Kontakt 23 entprechende, den Kapillarspalt 37 bildende Ausnehmungen eingearbeitet sind. Es sei angemerkt, daß auch schon eine einzelne Ausnehmung, sei sie im Oberteil 38 oder im plattenförmigen Kontakt 23 angeordnet, ausreichen würde, um einen Kapillarspalt 37 auszubilden. Die Ausnehmung im plattenförmigen Kontakt 23 kann gleichzeitig als Positionierhilfe im Zuge der Montage des Verdampfers 22 genutzt werden. Das Oberteil 38 ist mit dem plattenförmigen Kontakt 23 vorzugweise durch eine Klebeverbindung gefügt und besteht aus einem mit der Nikotinlösung 16 gut benetzbaren Werkstoff, vorzugsweise aus Leichtmetall oder aus einem benetzbaren Kunststoff; die Benetzbarkeit und im Übrigen auch die Verklebbarkeit von Kunststoffen kann durch eine Oberflächenaktivierung, beispielsweise durch eine Plasma-Behandlung mit Sauerstoff als Prozessgas erheblich verbessert werden.

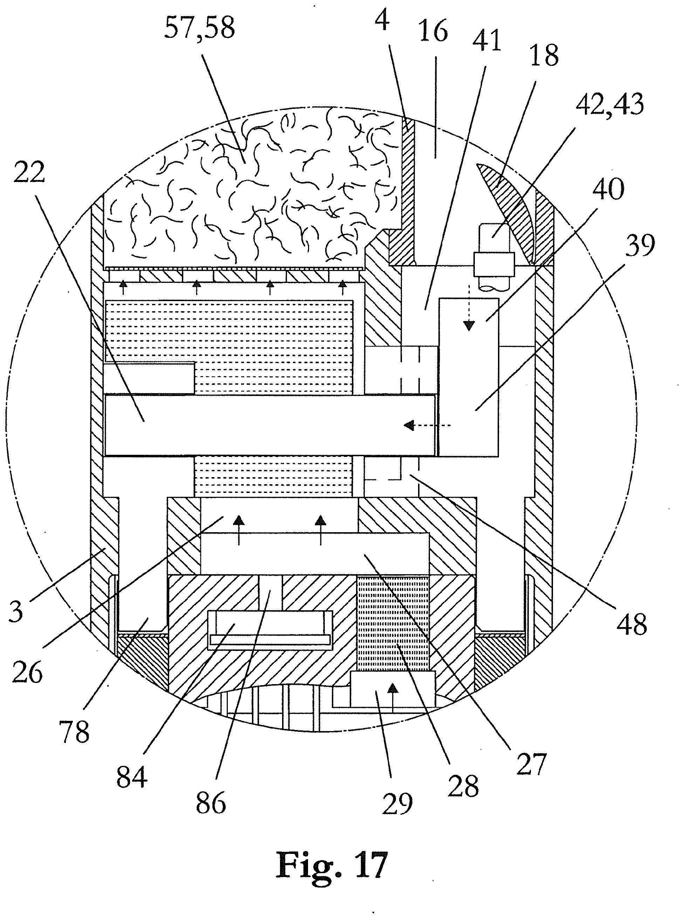

Weiter stromaufwärts wird der Kapillarspalt 37 durch zwei zueinander parallel und beabstandet angeordnete dünne Platten 39 gebildet (siehe Fig. 16), wobei eine Platte mit dem Oberteil 38 und die andere Platte mit dem plattenförmigen Kontakt 23 vorzugsweise durch eine Klebeverbindung verbunden sind. Die Platten 39 können beispielsweise aus einem Edelstahlband gestanzt werden. Wie Fig. 17-19 am besten zeigen, ragen die den Kapillarspalt 37 bildenden Platten 39 über einen Fortsatz 40 in ein Reservoir 41 hinein. Das Reservoir 41 schließt direkt an den Flüssigkeitsbehälter 4 an und ist von diesem nur durch den klappenartigen, öffenbaren Verschluß 18 getrennt. Der öffenbare Verschluß 18 wird mit Hilfe eines Stiftes 42 geöffnet. Der Stift 42 ist im Gehäuse 3 axial verschiebbar gelagert und besteht vorzugsweise aus Edelstahl. Ein erstes Ende 43 des Stiftes 42 ist gegen den öffenbaren Verschluß 18 gerichtet. Ein zweites Ende 44 ragt bei noch geschlossenem Verschluß 18 aus der äußeren Oberfläche des Gehäuses 3 fortsatzartig heraus. Das zweite Ende 44 des Stiftes 42 steht mit einem der beiden Kontaktelemente 20 des Inhalatorteils 1 in einer stößelartigen Wirkverbindung, indem das Kontaktelement 20 im Zuge der Kopplung der Inhalatorkomponente 2 mit dem Inhalatorteil 1 gegen das zweite Ende 44 des Stiftes 42 drückt, und der Stift 42 dadurch in das Gehäuse 3 verschoben wird. Die vom Kontaktelement 20 ausgeübte Druckkraft wird vom Stift 42 auf den öffenbaren Verschluß 18 übertragen. Der öffenbare Verschluß 18 weist auf seinem Umfang eine Materialschwächung 45 auf, welche so dimensioniert ist, daß sie bei Druckbeaufschlagung durch den Stift 42 gleich einer Sollbruchstelle über einen weiten Umfangsbereich aufreißt, jedoch auf einer Seite ein Scharnier 46 ausbildet. Auf diese Weise wird bewirkt, daß sich der öffenbare Verschluß 18 wie eine Klappe öffnet. Der Stift 42 weist nahe dem ersten Ende 43 eine Querschnittserweiterung 47 auf, welche in der Art eines Anschlages verhindert, daß der Stift aus dem Gehäuse 3 herausgleiten oder entnommen werden kann.

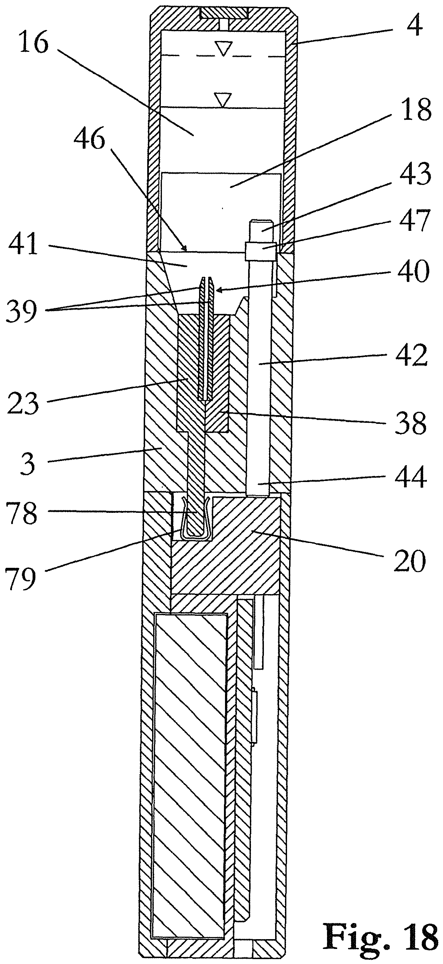

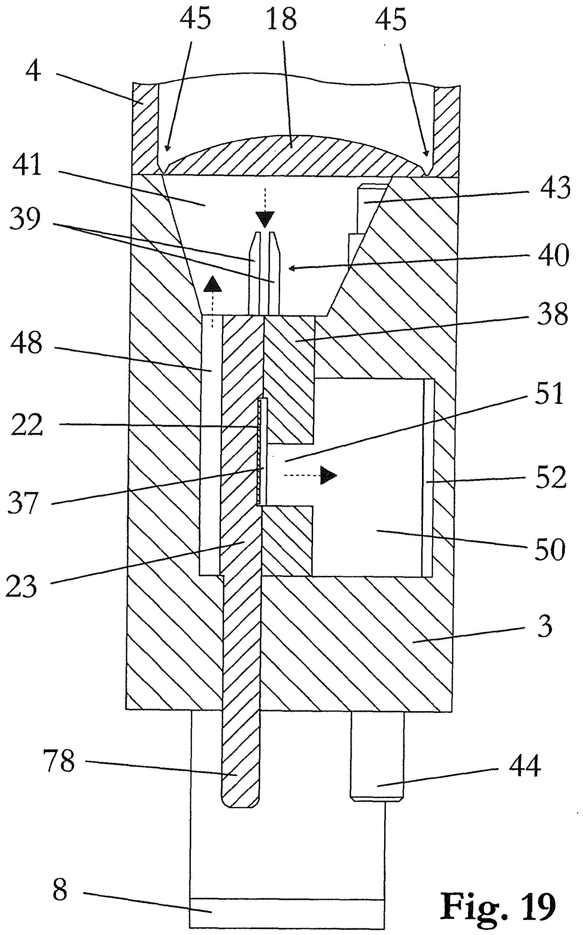

Die Versorgung des Verdampfers 22 mit der Nikotinlösung 16 soll im Folgenden zusammenfassend erläutert werden, wobei die Fig. 17 und Fig. 19 die Strömungsverhältnisse durch Pfeile veranschaulichen: im Zuge der Kopplung der Inhalatorkomponente 2 mit dem wiederverwendbaren Inhalatorteil 1 wird der klappenartige Verschluß 18 über den Stift 42 geöffnet, und in der Folge das Reservoir 41 vom flüssigen Material 16 unter Schwerkrafteinfluß geflutet. In Fig. 18 sind die Flüssigkeitsstände vor und nach dem Fluten eingezeichnet. Der Kapillarspalt 37 saugt die Nikotinlösung 16 über den Fortsatz 40 an und führt es dem Verdampfer 22 zu, wodurch schließlich der Docht vollständig mit dem flüssigen Material 16 infiltriert wird. Der durch die Platten 39 gebildete Fortsatz 40 soll vermeiden, daß sich im Mündungsbereich des Kapillarspalts 37 Gasblasen ansiedeln, welche die kapillare Kopplung behindern könnten. Desweiteren ist in den plattenförmigen Kontakt 23 ein Belüftungskanal 48 eingearbeitet, welcher das Reservoir 41 mit der Kammer 21 verbindet und einen Druckausgleich bewirkt. Auf diese Weise wird jede Portion flüssigen Materials 16, welche in den Kapillarspalt 37 gelangt, unmittelbar durch eine volumengleiche Portion Luft ersetzt. Schließlich ist in das Oberteil 38 ein Pufferspeicher 49 integriert - siehe Fig. 11 und Fig. 16. Der Pufferspeicher 49 besteht im gegenständlichen Ausführungsbeispiel aus parallel zueinander angeordneten Schlitzen 50, welche in das Oberteil 38 eingearbeitet sind. Die Schlitze 50 kommunizieren einerseits über Öffnungen 51 mit dem Kapillarspalt 37 und andererseits über einen Belüftungsspalt 52 mit der Kammer 21. Die Kapillarität der Schlitze 50 bewirkt, daß das flüssige Material 16 aus dem Reservoir 41 über den Kapillarspalt 37 und über die Öffnungen 51 in die Schlitze 50 strömt, wo es zwischengespeichert wird und vom Docht des Verdampfers 22 nach Bedarf wieder abgezogen werden kann, und zwar unabhängig von der Lage der Inhalatorkomponente 2.

Fig. 9-12 zeigen ferner eine in der Kammer 21 angeordnete erfindungsgemäße Kondensat-Drainage-und Speichereinrichtung bestehend aus zwei saugfähigen Schwämmen 53. Die grundsätzlichen Wirkungen der Kondensat-Drainage- und Speichereinrichtung wurden bereits früher näher erläutert. Die beiden Schwämme 53 sind plattenförmig ausgebildet und beabstandet und parallel zueinander angeordnet, wobei der flächige Verdampfer 22 von den beiden Schwämmen 53 beidseitig überdeckt wird. Zwischen den beiden Schwämmen 53 bildet sich ein Strömungskanal 54 aus, in welchem die Bildung des Dampf-Luft-Gemisches bzw. Kondensationsaerosols stattfindet. Der Hauptanteil der Kondensatrückstände wird an den, den Strömungskanal 54 bildenden Wandabschnitten 55 der Schwämme 53 abgeschieden und sofort von der offenen Porenstruktur der Schwämme aufgesaugt. Die Schwämme 53 sind an zwei gegenüberliegenden Wänden der Kammer 21 mittels einer Klebeverbindung befestigt, füllen den überwiegenden Teil der Kammer 21 aus und bestehen vorzugsweise aus einem hochporösen, formbeständigen und möglichst feinporigen Material. Bei Verwendung von grobporigem Material besteht nämlich die Gefahr, daß bei abrupten Bewegungen bzw. Beschleunigungen der Inhalatorkomponente 2 die Kapillarkräfte des Schwammmaterials nicht mehr ausreichen, um das flüssige Kondensat zurückzuhalten, und Teile des Kondensats aus den Schwämmen 53 herausgeschleudert werden. Als Schwammmaterial besonders geeignet erweisen sich Faserverbunde, gebildet aus thermisch oder mit Hilfe eines Bindemittels miteinander verbundenen Natur- oder Chemiefasern. Die Firma Filtrona Richmond Inc., www.filtronaporoustechnologies.com, ist auf die Herstellung solcher Faserverbunde spezialisiert, wobei sowohl mittels Triacetin gebundene Celluloseacetat-Fasern als auch thermisch gebundene Polyolefin- und Polyesterfasern verarbeitet weden. Die Inhalatorkomponente 2 sollte grundsätzlich nur gegen ein angemessenes Pfand an den Verbraucher abgegeben werden. Auf diese Weise wird sichergestellt, daß der Großteil der mit Nikotinrückständen kontaminierten Schwämme 53 umweltgerecht entsorgt wird.

Die Schwämme 53 sind zum Oberteil 38 und zu dem mit dem Oberteil 38 verbundenen plattenförmigen Kontakt 23 etwas beabstandet angeordnet, sodaß sich ein Spalt 56 bildet. Der Spalt 56 gewährleistet, daß der Belüftungskanal 48 sowie der Belüftungsspalt 52 ungehindert mit der Kammer 21 kommunizieren können. Die Schwämme 53 sind so zu dimensionieren, daß deren Porenvolumen die über die Nutzungsdauer der Inhalatorkomponente 2 gebildete Menge an Kondensatrückständen aufzunehmen vermag. Die Kondensatmenge hängt in erster Linie vom Ethanol- und Wasseranteil in der Nikotinlösung 16 sowie vom Luftdurchsatz durch die Lufteinlaßöffnung 26 bzw. durch den Strömungskanal 54 ab. Je weniger Luft durchgesetzt wird, umso weniger Dampf kann die Luft bis zur Sättigung aufnehmen.

Wie Fig. 9-10 und Fig. 12 zeigen, ist der Kammer 21 stromabwärts ein Kühler in Form einer Tabakfüllung 57 nachgeordnet, deren Poren vom gebildeten Dampf-Luft-Gemisch bzw. Kondensationsaerosol durchströmt werden. Die wesentlichen Wirkungen des Kühlers wurden bereits früher eingehend erläutert. Die Tabakfüllung 57 befindet sich in einem Füllraum 58, welcher strömungseingangsseitig durch eine Lochwand 59, strömungsausgangsseitig durch das Mundstück 5, und mantelseitig durch das Gehäuse 3 und durch eine Wand des Flüssigkeitsbehälters 4 begrenzt ist. Die Lochwand 59 stützt das Füllmaterial 57 und steift gleichzeitig das Gehäuse 3 aus. Die Lochwand 59 ist etwas beabstandet zu den Schwämmen 53 angeordnet - siehe Fig. 12. Dadurch wird erreicht, daß sich das aus dem Strömungskanal 54 austretende Dampf-Luft-Gemisch bzw. Kondensationsaerosol noch vor der Lochwand 59 gleichmäßig über den ganzen Querschnitt der Tabakfüllung 57 verteilen kann, und die Tabakfüllung 57 gleichmäßig durchströmt wird. Damit das Füllmaterial nicht aus den Löchern der Lochwand 59 austreten kann, ist zwischen der Tabakfüllung 57 und der Lochwand 59 ein erstes Drahtgewebe 60 angeordnet. Mundstückseitig wird die Tabakfüllung 57 durch ein zweites Drahtgewebe 61 begrenzt, welches verhindert, daß das Füllmaterial in den Mundstückkanal 62 oder gar in die Mundhöhle des Benutzers gelangen kann. Zwischen dem zweiten Drahtgewebe 61 und dem Mundstückkanal 62 bildet das Mundstück 5 eine Sammelkammer 63 aus, welche bewirkt, daß die Tabakfüllung 57 auch im Endabschnitt gleichmäßig durchströmt wird. Das zweite Drahtgewebe 61 ist vorteilhafterweise direkt am Mundstück 5 befestigt, z.B. auf dieses aufgeschmolzen. Im Zuge der Montage wird zuerst das erste Drahtgewebe 60 auf die Lochwand 59 aufgelegt. Danach wird eine vordefinierte Menge des Tabaks bzw. Füllmaterials 57 in den Füllraum 58 eingebracht, wobei das Befüllen auch mehrstufig erfolgen kann, und das Füllmaterial 57 nach jeder Teilbefüllung zwischenverdichtet wird. Auf diese Weise kann eine relativ homogene Fülldichte erzielt werden. Schließlich wird das Mundstück 5 montiert, und der Füllraum 58 verschlossen. In Prototypen wurden auf Basis von Feinschnitt-Tabak und einem Füllvolumen von etwa 7cm3 hinsichtlich der Qualität des auf diese Weise erzeugten Dampf-Luft-Gemisches bzw. Kondensationsaerosols, namentlich hinsichtlich der organoleptischen Eigenschaften desselben, ausgezeichnete Ergebnisse erzielt. Feinschnitt-Tabak zeichnet sich durch eine besonders kleine Schnittbreite von üblicherweise 0,3mm aus. Die kleine Schnittbreite bewirkt eine außerordentlich große Oberfläche der Tabakfüllung 57. Diese Bedingungen begünstigen sowohl den Wärmeaustauch als auch den Stoffaustausch zwischen dem Tabak und dem ihn durchsetzenden Dampf-Luft-Gemisch bzw. Kondensationsaerosol. Man unterscheidet bei Feinschnitt-Tabak zwei grundsätzlich verschiedene Typen bzw. Geschmacksrichtungen: helle American Blend Feinschnitte und dunkle Feinschnitte von meist würzigem Charakter. Beide Feinschnitte eignen sich zur Herstellung einer erfindungsgemäßen Tabakfüllung 57. Der Tabak kann zusätzlich gesoßt, gewürzt, aromatisiert oder/und parfümiert werden, wobei hier sämtliche von der Tabakindustrie verwendeten Rezepturen und angewendeten Verfahren übernommen werden können. In Prototypen wurden beispielsweise Menthol sowie mittels überkritischer CO2-Extraktion gewonnene Tabakaromaöle (vgl. Tabelle 1) und Kaffeeextrakt erfolgreich als aromatische Zusätze getestet. Tabakrauch-Kondensat oder eine flüchtige aromatische Fraktion desselben eignet sich ebenfalls als Aromazusatz. Um ein Entweichen von Aromastoffen aus der Inhalatorkomponente 2 zu verhindern, sollte diese in einer luftdichten Verpackung feilgeboten werden, und die Verpackung erst unmittelbar vor dem Gebrauch der Inhalatorkomponente 2 geöffnet werden. Auch noch nach der Kopplung der Inhalatorkomponente 2 mit dem Inhalatorteil 1 besteht die Möglichkeit, durch Verschließen der Mundstücköffnung beispielsweise mittels einer Kappe oder eines Stöpsels (nicht dargestellt) ein Entweichen von Aromastoffen zu unterdrücken.

Die Packungsdichte der Tabakfüllung 57 bestimmt den Strömungswiderstand, welchen das Füllmaterial 57 dem Dampf-Luft-Gemisch bzw. Kondensationsaerosol entgegensetzt; die Packungsdichte ist mit dem Strömungswiderstand der Strömungsdrossel 28 so abzustimmen, daß der resultierende Strömungswiderstand möglichst innerhalb des bereits genannten, bevorzugten Bereiches von 12-16mbar bei einem Luftdurchsatz von 1,05L/min liegt. Grundsätzlich ist es auch möglich, auf die Strömungsdrossel 28 ganz zu verzichten, und den gewünschten Strömungswiderstand allein durch die Tabakfüllung 57 zu erzeugen, indem deren Packungsdichte entsprechend erhöht wird. Generell sollte jedoch in diesem Zusammenhang beachtet werden, daß eine Filterwirkung der Tabakfüllung 57 unerwünscht ist; die in der Kammer 21 erzeugten Aerosolpartikel sollten die Tabakfüllung 57 möglichst ohne Verluste durchsetzen können. Die alternative Ausführungsvariante ohne Strömungsdrossel 28 hat außerdem Auswirkungen auf die sensortechnische Erfassung des Zugbeginns, welche Auswirkungen später noch näher erläutert werden.

Anstatt Tabak könnten alternativ auch andere Füllmaterialien zur Kühlung des Dampf-Luft-Gemisches bzw. Kondensationsaerosols Verwendung finden, vor allem, wenn die Verwendung von Tabak als Arzneimittel aus scheinheiligen gesundheitspolitischen Gründen abgelehnt wird. Der Anmelder ist allerdings davon überzeugt, daß der Umstieg von der Zigarette auf die erfindungsgemäße Inhalatorkomponente durch die Verwendung von Tabak als Füllmaterial 57 wesentlich erleichtert wird. Es erscheint aus diesem Grund auch zweckdienlich, das Vorhandensein des Tabaks in der Inhalatorkomponente 2 durch geeignete Mittel hervorzuheben. Tabakfreie, "rein künstliche" Zigaretten-Ersatzprodukte würden beim Durchschnittsverbraucher, insbesondere bei ehemaligen Zigarettenrauchern, aller Wahrscheinlichkeit nach nicht dieselbe Akzeptanz finden. Der Tabak wird in der Inhalatorkomponente 2 weder verbrannt, noch wird er nennenswert erwärmt, so daß von ihm keine gesundheitsschädlichen Wirkungen ausgehen. Als alternatives Füllmaterial 57 bietet sich grundsätzlich jedes Porenmaterial an, sofern dieses für die Aerosolpartikel weitgehend durchlässig und unter den gegebenen Einsatzbedingungen weitestgehend chemisch inert ist. Ein Filtermaterial, welches eine Abscheidung der Aerosolpartikel bewirken würde, scheidet von vornherein als Porenmaterial aus. Das Porenmaterial muß also hinreichend grob- bzw. weitporig sein, um sich als Füllmaterial 57 zur Kühlung des Dampf-Luft-Gemisches bzw. Kondensationsaerosols zu qualifizieren. Als alternatives Füllmaterial 57 eignen sich beispielsweise Regeneratormaterialien, wie Füllungen aus Metallwolle oder Metallspänen, oder Schüttungen aus metallischem oder keramischem Granulat, wobei vorteilhafterweise das Granulat selbst eine poröse Struktur aufweisen sollte. Füllungen aus Natur- oder Chemiefasern kommen ebenfalls in Betracht, sofern diese hinreichend weitporig und weitestgehend chemisch inert sind. Zur Aromatisierung des alternativen Füllmaterials 57 eignet sich zuallererst Tabak, zum Beispiel in disperser Form als Soße zugesetzt. Verbietet sich auch die Verwendung von Tabak als Zusatz zur Aromatisierung, muß auf alternative Rezepturen und Verfahren ausgewichen werden, welche allerdings kaum das natürliche Tabakaroma in seiner ganzen Komplexität je nachbilden werden können.

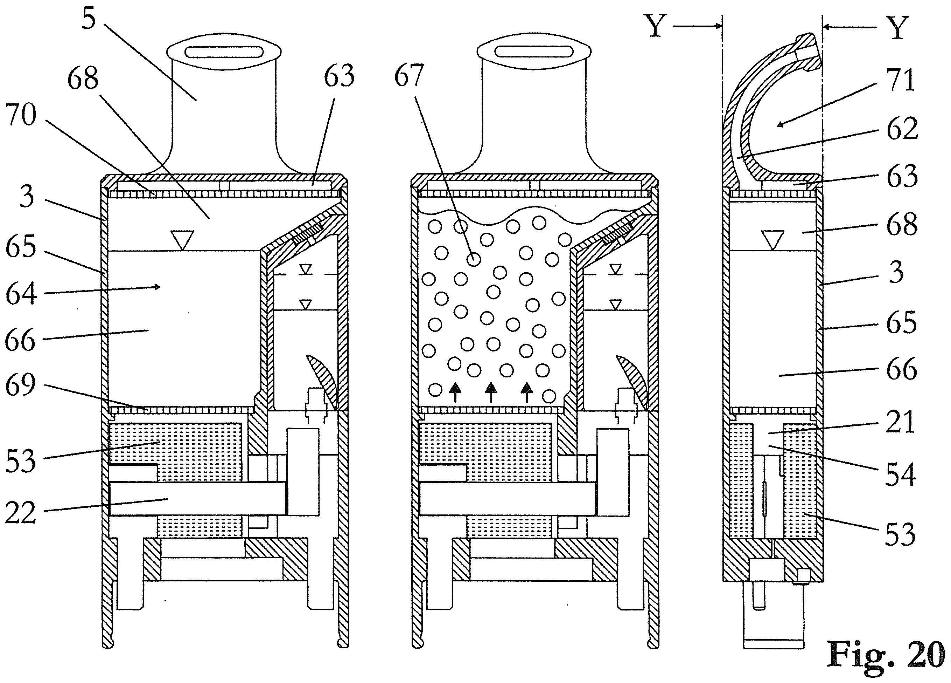

Fig. 20 zeigt eine alternative Ausführungsform des Kühlers. Demnach ist der Kühler als Flüssigkeitskühler 64 ausgebildet. Der Flüssigkeitskühler 64 besteht aus einem vom Gehäuse 3 ausgebildeten Behälter 65, welcher der Kammer 21 stromabwärts nachgeordnet ist. Der Behälter 65 beinhaltet eine Kühlflüssigkeit 66, welche vom gebildeten Dampf-Luft-Gemisch bzw. Kondensationsaerosol in Form von Gasblasen 67 durchströmt wird. Die Kühlflüssigkeit 66 besteht im Wesentlichen aus Wasser oder/und Ethanol. Der Kühlflüssigkeit 66 können optional Konservierungsmittel zugesetzt sein. Der Behälter 65 ist nicht vollständig mit Kühlflüssigkeit gefüllt. Vielmehr ist ein Pufferraum 68 vorgesehen, welcher es der Kühlflüssigkeit 66 gestattet, sich während der Durchströmug des Dampf-Luft-Gemisches bzw. Kondensationsaerosols ungehindert auszudehnen. Für das Volumen Vp des Pufferraums 68 gilt die Bedingung: Vp>Vbl (Vbl ... Gesamtvolumen aller Gasblasen während der Durchströmung).

Der Behälter 65 wird mantelseitig durch das Gehäuse 3 gebildet. Stirnseitig kommuniziert der Behälter 65 mit der Kammer 21 einerseits und mit dem Mundstück 5 andererseits über eine Flüssigkeitssperre in Form zweier semipermeabler Membranen 69 und 70. Solche Membranen können beispielsweise von der Firma Porex Technologies, www.porex.com, in Form von porösen, gesinterten Kunststoff-Membranen bezogen werden. Porex Technologies fertigt solche Membranen unter anderem aus naturhydrophoben sowie extrahydrophobisierten Kunststoffen wie PTFE, PE und PP in Dicken ab 0,5mm und mit Porendurchmessern von 10-150µm. Die aus PTFE gefertigten Membranen werden unter der Bezeichnung Mupor^{®} vertrieben. Der Porendurchmesser sollte im Bereich 50-150µm gewählt werden; noch kleinere Porendurchmesser führten zu unzulässig hohen Strömungs- bzw. Zugwiderständen. Die kammerseitige Membran 69 dient gleichzeitig zur Einleitung und Verdüsung des Dampf-Luft-Gemisches bzw. Kondensationsaerosols in die bzw. in der Kühlflüssigkeit 66. Die kammerseitige Membran 69 wirkt insofern wie ein Düsenboden oder ähnlich einer Fritte in einer Waschflasche. Die kammerseitige Membran 69 ist etwas beabstandet zu den Schwämmen 53 angeordnet. Dadurch wird erreicht, daß die Membran 69 vom aus dem Strömungskanal 54 zuströmenden Dampf-Luft-Gemisch bzw. Kondensationsaerosol über ihren gesamten Querschnitt annähernd gleichmäßig beaufschlagt wird. Zwischen der mundstückseitigen Membran 70 und dem Mundstückkanal 62 bildet das Mundstück 5 wieder eine Sammelkammer 63 aus, welche bewirkt, daß auch die Membran 70 vom mittlerweile gekühlten Dampf-Luft-Gemisch bzw. Kondensationsaerosol über ihren gesamten Querschnitt beaufschlagt und durchsetzt wird. Auf diese Weise kann der Sperreffekt der Membran 70 großflächig genutzt werden, und gleichzeitig der Strömungswiderstand auf einem niedrigen Niveau gehalten werden.

Für den funktionsgerechten Betrieb des Flüssigkeitskühlers ist es wesentlich, daß der Benutzer den Behälter 65 annähernd senkrecht hält, sodaß die Kühlflüssigkeit 66 in Bezug auf die Membran 69 allseits einen annähernd konstanten Flüssigkeitspegel aufweist. Nur so kann gewährleistet werden, daß die Gasblasen 67 die Kühlflüssigkeit 66 über den gesamten Querschnitt relativ gleichmäßig durchsetzen, und das Potenzial der Flüssigkeitskühlung voll ausgeschöpft wird. Die spezielle Anordung und gekrümmte Form des Mundstückes 5 soll den Benutzer unterstützen, den Behälter 65 in der korrekten Lage zu halten. Die durch die Krümmung des Mundstückes 5 gebildete Einbuchtung 71 dient zur Aufnahme der Unterlippe des Benutzers. Konstruktiv bemerkenswert ist ferner, daß das Mundstück 5 trotz seiner beachtlichen Krümmung über keine der Begrenzungsebenen Y des als Flachgehäuse ausgebildeten Gehäuses 3 hinausragt. Der Effekt dieser Anordnung wurde bereits früher erläutert.

Zur Aromatisierung des Flüssigkeitskühlers 64 können der Kühlflüssigkeit 66 optional Tabak oder/und Aromastoffe zugesetzt werden. Hierbei ist es nicht zwingend erforderlich, daß der Tabak oder/und die Aromastoffe in der Kühlflüssigkeit 66 löslich sind. Der Tabak oder/und die Aromastoffe können auch in der Kühlflüssigkeit 66 dispergiert oder als Sediment vorliegen, oder auch nur auf der Kühlflüssigkeitsoberfläche aufschwimmen. Die die Kühlflüssigkeit 66 durchsetzenden Gasblasen 67 erzeugen eine erhebliche Strömungsturbulenz, welche nicht nur den Wärmeaustausch sondern auch den Stoffaustausch begünstigt. In Prototypen hat sich die Zugabe von gemahlenem Tabak, wie er beispielsweise in Schnupftabak Verwendung findet, bewährt. Die Tabakpartikel liegen in der Kühlflüssigkeit 66 als Suspension vor, wobei sich ein Großteil der Partikel auf der Membran 69 als Sediment absetzt. Während eines Zuges wird das Sediment durch die aufsteigenden Gasblasen 67 aufgewirbelt und wie in einem Wirbelbett umgewälzt. In Prototypen wurden außerdem Menthol sowie mittels überkritischer CO2-Extraktion gewonnene Tabakaromaöle (vgl. Tabelle 1) und Kaffeeextrakt erfolgreich als aromatische Zusätze getestet. Um ein Entweichen von Aromastoffen aus der Inhalatorkomponente 2 zu verhindern, sollte diese in einer luftdichten Verpackung feilgeboten werden, und die Verpackung erst unmittelbar vor dem Gebrauch der Inhalatorkomponente geöffnet werden. Auch noch nach der Kopplung der Inhalatorkomponente 2 mit dem Inhalatorteil 1 besteht die Möglichkeit, durch Verschließen der Mundstücköffnung beispielsweise mittels einer Kappe oder eines Stöpsels (nicht dargestellt) ein Entweichen von Aromastoffen zu unterdrücken.

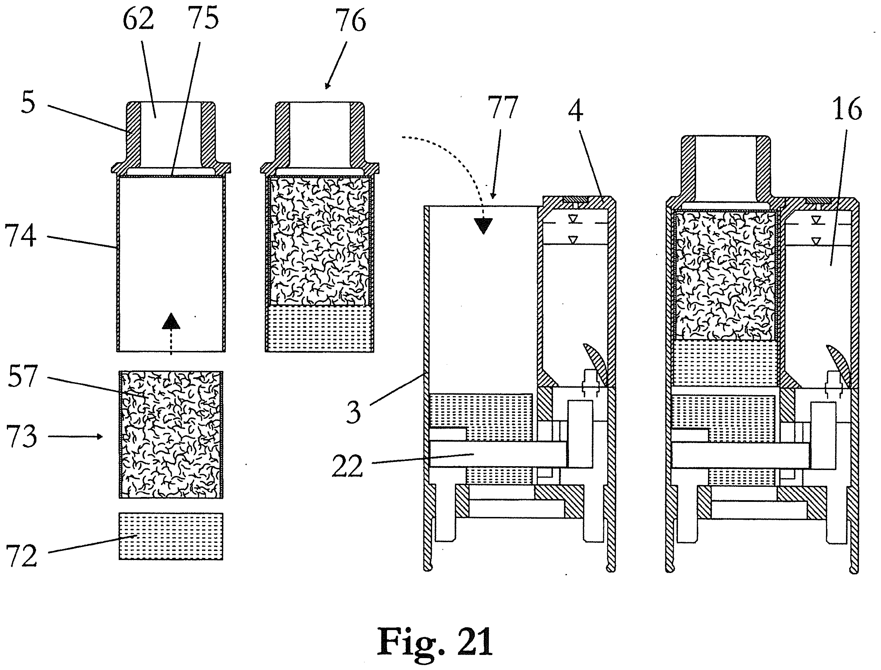

Fig. 21 zeigt eine weitere alternative Ausführungsform des Kühlers. Danach ist der Kühler zweistufig aufgebaut und besteht aus einem Vorkühler 72 und einer diesem nachgeordneten Tabakfüllung 57. Der Vorkühler 72 bildet die erste Kühlerstufe und hat die Aufgabe, das Dampf-Luft-Gemisch bzw. Kondensationsaerosol möglichst effektiv und weitgehend vorzukühlen, bevor dieses die Tabakfüllung 57 erreicht. Ferner schirmt der Vorkühler 72 die Tabakfüllung 57 von der Kammer 21 ab, so daß die Tabakfüllung 57 von den Kondensationsvorgängen in der Kammer 21 nicht tangiert wird. Diese Bedingungen führen dazu, daß die Tabakfüllung 57 weniger mit Lösungsmittelkondensat beladen wird. Eine allzu große Kondensatbeladung der Tabakfüllung 57 kann die aromatisierende Wirkung derselben nachteilig beeinflussen. Der Vorkühler 72 kann beispielsweise aus einem Synthetikfaservlies bestehen. Die Firma Freudenberg Vliesstoffe KG, www.freudenberg-filter.com, bietet ein solches Material in Form von Matten/ Platten unter der Bezeichnung Viledon^{®}-Filtermatten an. Das Material kann nach Kundenspezifikation angefertigt werden. Insbesondere können die Materialeigenschaften so abgestimmt werden, daß das Endprodukt für die feinen Partikel des erzeugten Kondensationsaerosols weitestgehend durchlässig ist. Die Matten/ Platten werden aus Polyolefinfasern (PE, PP) oder Polyesterfasern gefertigt und können durch Stanzen weiterverarbeitet werden. Die Tabakfüllung 57 liegt als vorfabrizierte Packung vor und besteht aus einem papierumwickelten Tabakstrang 73, welcher vorteilhafterweise aus einem Endlosstrang gewonnen wird. Der Tabakstrang 73 sowie der Vorkühler 72 sind in einen Hohlzylinder 74 eingeschoben. Der Hohlzylinder 74 ist Teil des Mundstückes 5. Mundstückseitig wird die Tabakfüllung 57 durch ein Drahtgewebe 75 begrenzt, welches verhindert, daß das Füllmaterial in den Mundstückkanal 62 oder gar in die Mundhöhle des Benutzers gelangen kann. Der Vorkühler 72 und der Tabakstrang 73 bilden mit dem Mundstück 5 eine bauliche Einheit 76, welche über die Gehäuseöffnung 77 in das Gehäuse 3 der Inhalatorkomponente 2 einsetzbar ist. Diese Anordnung erlaubt es dem Benutzer, die Mundstück-Kühler-Einheit 76 unabhängig von den übrigen Bestandteilen der Inhalatorkomponente 2 auszuwechseln. Dies ermöglicht es, die Inhalatorkomponente 2 mit einem vergleichsweise großen Flüssigkeitsbehälter 4 mit einem entsprechend großen Vorrat an Nikotinlösung 16 auszustatten, welcher Vorrat bei durchschnittlicher Konsumation bis zu einer Woche und darüber hinaus reichen kann, während die Mundstück-Kühler-Einheit 76 nicht zuletzt auch aus hygienischen Gründen vorzugsweise täglich ersetzt wird.

Der Tabakstrang 73 kann optional zusätzlich aromatisiert werden. Die aromatischen Zusätze können, sofern sie in flüssiger Form vorliegen, über eine oder mehrere Injektionsnadeln in den Tabakstrang 73 eingebracht werden. Zähflüssige und feste Aromastoffe können zu diesem Zweck zuvor in einem geeigneten Lösungsmittel verdünnt bzw. gelöst werden. Typische aromatische Zusätze sind mittels überkritischer CO2-Extraktion gewonnene Tabakaromaöle (vgl. Tabelle 1), Kaffeeextrakt, Tabakrauch-Kondensat oder eine flüchtige aromatische Fraktion eines Tabakrauch-Kondensats, um nur einige Beispiele zu nennen. Der Tabakstrang 73 kann ferner wie Zigaretten mentholisiert werden, wobei analoge Verfahren zur Anwendung kommen können. Die Mundstück-Kühler-Einheit 76 ist nach der Aromatisierung sofort luftdicht zu verschließen, indem sie beispielsweise in eine Schutzfolie eingeschweißt wird. Das Einschweißen erfolgt vorzugsweise unter Schutzgasatmosphäre, z.B. unter Stickstoff; auf diese Weise kann einer Oxidation sowie einem mikrobiellen Befall durch Bakterien und Schimmelpilze vorgebeugt werden.

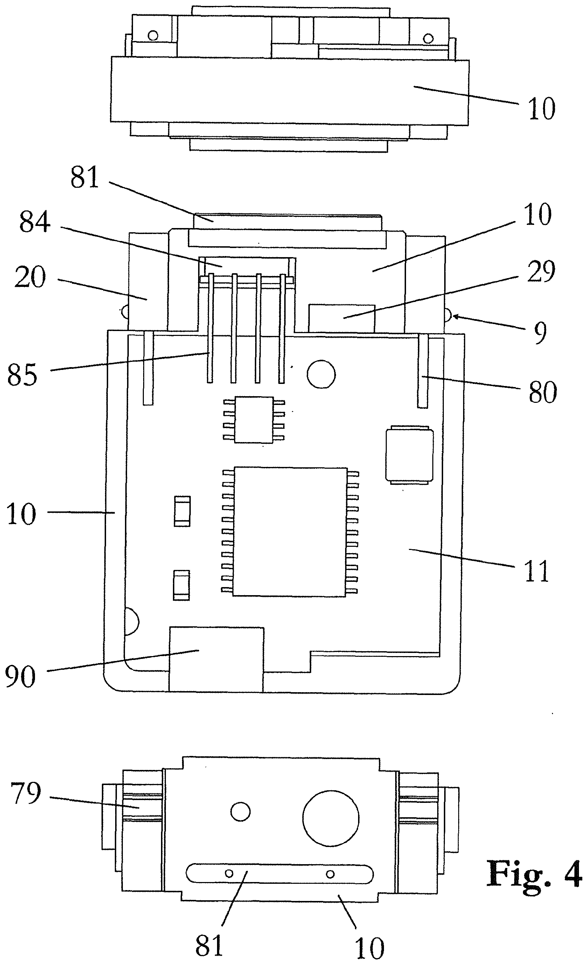

Im Folgenden sollen noch weitere, allgemeine Bestandteile des Inhalators näher beschrieben werden, soweit diese für das Verständnis und die Ausführbarkeit der Erfindung relevant erscheinen: wie Fig. 6, Fig. 9 und Fig. 18 zeigen, ragen die plattenförmigen Kontakte 23 der auswechselbaren Inhalatorkomponente 2 aus der äußeren Oberfläche des Gehäuses 3 in Form zweier Steckkontakte 78 heraus. Die Steckkontakte 78 bilden mit korrespondierenden Federkontakten 79 im Zuge der Kopplung der Inhalatorkomponente 2 mit dem Inhalatorteil 1 elektrische Kontakte aus, über welche dem elektrischen Widerstandsheizelement die elektrische Energie zur Verdampfung der Nikotinlösung 16 zugeführt wird. Die Federkontakte 79 sind Teil der Kontaktelemente 20 und vorzugsweise durch eine Schweißverbindung mit diesen verbunden - siehe auch Fig. 4-5. Die Kontaktelemente 20 bestehen aus einem metallischen Kontaktwerkstoff und können beispielsweise von der Firma Ami Doduco GmbH, www.amidoduco.com gefertigt werden. Für den Fall, daß für die plattenförmigen Kontakte 23 aus den bereits genannten Gründen dasselbe oder ein ähnliches Material verwendet wird wie für das Heizelement des Verdampfers, z.B. Edelstahl, ist es aufgrund der unzureichenden elektrischen Leitfähigkeit dieses Materials erforderlich, die plattenförmigen Kontakte 23 zumindest im Bereich der Steckkontakte 78 beispielsweise galvanisch mit einer Leitschicht aus Gold, Silber, Palladium oder/und Nickel zu überziehen, wodurch der elektrische Kontaktwiderstand wesentlich herabgesetzt wird. Die Kontaktelemente 20 beziehen die elektrische Energie über zwei Drähte 80, welche die Kontaktelemente 20 mit der Platine 11 verbinden - siehe Fig. 4-5. Die Drähte 80 sind vorzugsweise beidseitig durch eine Lötung befestigt. Zusammenfassend sei nochmals darauf hingewiesen, daß die Kontaktelemente 20 drei verschiedene Aufgaben erfüllen: erstens übertragen sie, wie zuvor gerade beschrieben, die elektrische Energie von der Platine 11 auf die plattenförmigen Kontakte 23. Zweitens bilden sie seitliche Rastnasen 9 aus, welche mit den Schnapphaken 8 des Gehäuses 3 zusammenwirken, wodurch die Schnappverbindung zwischen der Inhalatorkomponente 2 und dem Inhalatorteil 1 verwirklicht wird. Und drittens bildet eines der beiden Kontaktelemente 20 einen Anschlag für den Stift 42 aus, wodurch die stößelartige Wirkverbindung zur Öffnung des Flüssigkeitsbehälters 4 hergestellt wird.

Zur lagegenauen Kopplung der Inhalatorkomponente 2 mit dem Inhalatorteil 1 ist eine Positioniereinrichtung vorgesehen, welche aus einem am Trägergehäuse 10 angeordneten Zentriervorsprung 81, und einer mit diesem korrespondierenden und am Gehäuse 3 angeordneten Zentrierausnehmung 82 besteht - siehe Fig. 3, Fig. 6, Fig. 10 und Fig. 12. Der Zentriervorsprung 81 weist zwei Entlüftungslöcher 83 auf, welche die Zentrierausnehmung 82 im Zuge der Kopplung entlüften.

Einen wesentlichen Bestandteil des erfindungsgemäßen Inhalators bildet der Drucksensor 84 - siehe Fig. 8 und Fig. 17. Der Drucksensor 84 hat die Aufgabe, den Beginn eines Zuges zu detektieren, woraufhin der elektrische Schaltkreis 11 die Zufuhr der elektrischen Energie zum Heizelement des Verdampfers 22 aktiviert, und die Verdampfung der im Docht gespeicherten Nikotinlösung 16 einsetzt. Der Drucksensor 84 ist in das Trägergehäuse 10 eingeklebt, und seine elektrischen Anschlüsse oder Pins 85 sind direkt auf der Platine 11 verlötet. Der Drucksensor 84 kommuniziert über eine Bohrung 86 mit der Plenumkammer 27 und mißt bzw. überwacht den Druck in der Plenumkammer 27 - siehe Fig. 17. Als Drucksensor 84 eignet sich beispielsweise der Typ CPCL04GC des Herstellers Honeywell Inc., www.honeywell.com mit einem Meßbereich von +/-10mbar. Der genannte Sensor besteht im Wesentlichen aus einer Nullpunkt-kalibrierten und Temperatur-kompensierten Meßbrücke und kann auf der Platine 11 folgendermaßen beschaltet werden: der negative Sensorausgang wird über einen hochohmigen Widerstand mit einem definierten Widerstandswert - z.B. 2,2MOhm - auf Masse gelegt, wodurch das Ausgangs- oder Meßsignal des Drucksensors 84 geringfügig verzerrt wird, oder anders formuliert, der Offset der Meßbrücke auf einen definierten Wert kalibriert wird. Durch die Verzerrung bzw. durch den Offset wird eine Schaltschwelle vorgegeben, welche einem bestimmten Druck-Schwellwert entspricht. Das auf diese Weise aufbereitete Meßsignal wird auf den Eingang eines als Komparator geschalteten Präzisions-Operationsverstärkers 87 - z.B. des Typs LTC1049CS8 des Herstellers Linear Technology Inc., www.linear.com gelegt. Aus dieser Beschaltung resultiert ein Ausgangssignal, welches den Zugbeginn in digitaler Form überaus schnell und exakt abbildet. Der Drucksensor 84 eignet sich als Sensor zur Erfassung des Zugbeginns freilich nur, wenn stromaufwärts von der Plenumkammer 27 eine Strömungsdrossel 28 angeordnet ist. In diesem Fall tritt in der Plenumkammer 27 im Verlauf eines Zuges bezogen auf die Umgebung ein Unterdruck auf, welcher typischerweise im Bereich 0-50mbar liegt. Der Druckverlauf hat näherungsweise eine Glockenform. Der Zugbeginn kann auf einfache Weise detektiert werden, indem ein Druck-Schwellwert, wie zuvor beschrieben, vorgegeben wird, welcher ständig mit dem tatsächlich gemessenen Druck verglichen wird. Der Zugbeginn kann als das erstmalige Überschreiten des Druck-Schwellwertes definiert werden. Für den Druck-Schwellwert wird zweckmäßigerweise ein Wert im Bereich 0,2-5mbar gewählt. Je kleiner der Druck-Schwellwert gewählt wird, umso schneller spricht die Zugerkennung an. Eine untere Grenze wird durch die Spezifikationen des jeweiligen verwendeten Drucksensors und Operationsverstärkers gezogen.

Ist im Inhalator keine Strömungsdrossel 28 vorgesehen, und wird der gewünschte Strömungswiderstand bzw. Zugwiderstand allein durch den durchströmten Kühler erzeugt, so herrscht in der Plenumkammer 27 praktisch Umgebungsdruck. In diesem Fall ist es zweckmäßiger, den Zugbeginn mittels eines im Querkanal 29 angeordneten Strömungssensors (nicht dargestellt) zu detektieren, dessen Anschlüsse oder Pins vorteilhafterweise wieder direkt auf der Platine 11 verlötet sein können. Als Strömungssensor eignet sich vorzugsweise ein Thermistor, beispielsweise des Typs GR015 des Herstellers Betatherm Corporation, www.betatherm.com. Der Thermistor ist auf der Platine 11 zu einer Meßbrücke (nicht dargestellt) verschaltet. Die Meßbrücke beinhaltet zur Temperaturkompensation einen typgleichen zweiten Thermistor und wird mittels Präzisionswiderständen auf einen definierten Offset-Schwellwert kalibriert. Das Ausgangssignal der Meßbrücke wird sodann wieder auf den Eingang eines als Komparator geschalteten Operationsverstärkers gelegt. Im Gleichgewichtszustand befinden sich beide Thermistoren auf gleichem Temperaturniveau - typischerweise im Bereich 80-200°C, abhängig von der dissipierten Leistung. Sobald nun ein Benutzer am Mundstück 5 zieht, strömt Luft durch den Querkanal 29. Die Luft kühlt den Thermistor ab, wodurch dessen Widerstand steigt. Die Widerstandsänderung wird von der Meßbrücke verarbeitet. In dem Moment, wo das Ausgangssignal der Meßbrücke den Nullpunkt durchschreitet, kippt der Komparator und gibt ein den Zugbeginn kennzeichnendes digitales Signal aus.

Die Weiterverarbeitung des vom Druck- bzw. Strömungssensor und dessen Beschaltung ausgegebenen Signals erfolgt vorzugsweise in einem integrierten Schaltkreis 88 - siehe Fig. 8. Der integrierte Schaltkreis 88 kann auch ein Mikroprozessor sein. Der integrierte Schaltkreis 88 verarbeitet einen Großteil aller elektrischen Signale des Inhalators und führt die für den Betrieb des Inhalators wesentlichen Steuer-Operationen aus. Eine zentrale Steuer-Operation stellt die Zufuhr der elektrischen Energie zum Heizelement des Verdampfers 22 dar. Die elektrische Energie wird vom Energiespeicher 12 geliefert. Basierend auf dem heutigen Stand der Technik bieten sich Lithium-Polymer- und Lithium-Ionen-Zellen aufgrund ihrer hohen Energie- und Leistungsdichte in besonderer Weise als Energiespeicher 12 an. Im Fall von metallischen Heizelementen findet man bereits mit einer einzelnen Lithium-Polymer- oder Lithium-Ionen-Zelle mit einer Leerlauf-oder Nennspannung von etwa 3,7V das Auslangen. Die Regelung der Energie- und Leistungszufuhr zum Heizelement des Verdampfers 22 kann in einfacher Weise dadurch erfolgen, daß die Batteriespannung über die Dauer der Energiezufuhr mit variablem Aussteuerungsgrad zerhackt wird, und die daraus resultierende Nutzspannung am Heizelement angelegt wird. Die resultierende Nutzspannung ist ein Rechteck-Signal mit variablem Tastverhältnis (Duty Cycle). Die Amplitude des Rechteck-Signals entspricht, wenn man von geringen Spannungsverlusten einmal absieht, der Batteriespannung. Die eigentliche Zerhackung erfolgt vorzugsweise mittels eines Leistungs-MOSFETs 89, z.B. des Typs IRF6635 des Herstellers International Rectifier, www.irf.com, welcher dazu geeignet ist, sehr hohe Ströme bei minimalem Drain-Source-Durchlasswiderstand zu schalten. Der integrierte Schaltkreis 88 steuert dabei das Gate des Leistungs-MOSFETs 89. Eine sehr einfache Regelungsstrategie, welche sich im Übrigen auch in erfindungsgemäßen Prototypen bewährt hat, besteht darin, die Dauer der Energiezufuhr in zwei Perioden zu unterteilen - in eine Aufheizperiode und eine daran anschließende Verdampfungsperiode. Im intermittierenden, zugsynchronen Betrieb des Inhalators orientiert sich die Dauer der Energiezufuhr an der Dauer eines Zuges. Es kann von einer durchschnittlichen Zugdauer von etwa 2,1sec (+/-0,4sec) ausgegangen werden. Derselbe Wert gilt in etwa auch für Zigaretten. Berücksichtigt man, daß auch nach Abschalten der Energiezufuhr wegen der im Verdampfer 22 noch gespeicherten Wärme zu einem gewissen Grad eine Nachverdampfung stattfindet, erscheint es zweckmäßig, die Dauer der Energiezufuhr etwas kürzer zu wählen, z.B. einen Wert im Bereich 1,5-1,8sec. Während der ersten der zuvor genannten zwei Perioden - der Aufheizperiode - wird der Verdampfer 22 samt der im Docht gespeicherten Nikotinlösung 16 durch das Heizelement aufgeheizt. Die Verdampfung der Nikotinlösung 16 setzt erst ein, wenn die Temperatur des Verdampfers 22 etwa den Siedebereich der niedrig siedenden Fraktionen der Nikotinlösung 16 erreicht hat. Die Aufheizperiode sollte daher möglichst kurz sein. Insofern liegt es nahe, die Batteriespannung in dieser Periode unzerhackt bzw. mit einem Aussteuerungsgrad oder Duty Cycle von 100% an das Heizelement weiterzugeben. Die Dauer der Aufheizperiode hängt vor allem von den Spezifikationen des Verdampfers 22 und von der Menge und Zusammensetzung der Nikotinlösung 16 ab und sollte möglichst <0,5sec sein. In der anschließenden zweiten Periode - der Verdampfungsperiode - wird der Aussteuerungsgrad wesentlich zurückgenommen, und es erfolgt die eigentliche Verdampfung der Nikotinlösung 16. Die zugeführte Energie wird in dieser zweiten Periode primär zur Verdampfung der Nikotinlösung 16 und sekundär zur Deckung von Energieverlusten verwendet. Durch entsprechende Wahl des Aussteuerungsgrades kann die Verdampfungsleistung und damit auch die pro Zug oder Inhalation verdampfte Menge der Nikotinlösung 16 in gewissen Grenzen gesteuert werden. Eine obere Grenze ist durch das Auftreten einer Siedekrise sowie durch ein lokales Austrocknen und Überhitzen des Dochts gesetzt. Durch eine Zurücknahme bzw. Drosselung des Aussteuerungsgrades kann hingegen einer thermischen Zersetzung des flüssigen Materials 16 entgegengewirkt werden.

Die soeben beschriebene Regelungsstrategie kann beliebig erweitert und verfeinert werden: zum Beispiel kann es sinnvoll sein, auch den Zustand der Batterie 12 in der Regelungsstrategie zu berücksichtigen, da die Batteriespannung mit zunehmender Entladung und zunehmendem Alter der Batterie vor allem unter Last deutlich sinkt. Diesem Effekt kann mit einer Erhöhung des Aussteuerungsgrades begegnet werden. Um diese Korrektur auch in der Aufheizperiode vornehmen zu können, ist es zweckmäßig, die Batteriespannung einer neuen, geladenen Batterie nicht wie früher vorgeschlagen zu 100%, sondern z.B. nur zu 80% auszusteuern, sodaß noch genügend Spielraum für eine Anpassung bleibt.

Die Steuerung der Energiezufuhr zum Heizelement des Verdampfers 22 erfordert außerdem verschiedene Hilfs-Operationen: beispielsweise muß vorgesehen werden, daß die Energiezufuhr nach dem Ende eines Verdampfungszyklus nicht gleich nochmals aktiviert werden kann. Vielmehr ist eine Wartezeit einzuhalten, welche der Nikotinlösung 16 genügend Zeit läßt, den Docht von neuem vollständig zu infiltrieren. Die mindestens erforderliche Wartezeit hängt von den jeweiligen Spezifikationen des Verdampfers 22 sowie von der Zähigkeit des flüssigen Materials 16 ab. In Prototypen konnte gezeigt werden, und Berechnungen bestätigen, daß bei entsprechender Auslegung eine vollständige Infiltration des Dochts in weniger als 10sec erzielt werden kann. Eine verbindliche Wartezeit in dieser Größenordnung sollte von den meisten Benutzern toleriert werden, vor allem wenn man berücksichtigt, daß im Fall der Zigarette das Intervall zwischen zwei Zügen durchschnittlich 25sec beträgt. Eine solche Wartezeit ist gleichfalls nach dem Ankoppeln einer neuen Inhalatorkomponente 2 an das Inhalatorteil 1 einzuhalten. Eine andere Hilfs-Operation besteht darin, daß die Energiezufuhr zum Heizelement des Verdampfers 22 sofort abgebrochen wird, wenn der Benutzer den Zug vorzeitig abbricht. Auf diese Weise wird verhindert, daß in der Kammer 21 unnötig Dampf gebildet wird.

Der integrierte Schaltkreis 88 führt vorzugsweise noch weitere Steuer-Operationen aus. Als Beispiele seien genannt: Kommunikation mit dem Benutzer (Benutzer-Schnittstelle), Dosage Recording, Identifizierung der verwendeten Inhalatorkomponente 2, Identifizierung des Benutzers, Zell- und Lademanagement der Batterie 12. Die Umsetzung aller Steuer-Operationen in einen Schaltplan kann von jedem auf diesem Gebiet versierten Fachmann unter Anwendung bekannter Methoden geleistet werden, und soll daher in diesem Rahmen auch nicht weiter erörtert werden.

Abschließend sei nochmals die Funktions- und Betriebsweise des Inhalators zusammenfassend dargestellt: der Benutzer macht eine neue Inhalatorkomponente 2 einsatzbereit, indem er sie über die Schnappverbindung 8, 9 mit dem wiederverwendbaren Inhalatorteil 1 koppelt. Das Öffnen des Flüssigkeitsbehälters 4 erfolgt synchron zur Kopplung mit dem Inhalatorteil 1 mittels des Stiftes 42 im Zusammenwirken mit dem Kontaktelement 20 (siehe Fig. 18), woraufhin das als Fortsatz 40 ausgebildete Ende des Kapillarspalts 37 mit der Nikotinlösung 16 benetzt wird. Der Kapillarspalt 37 übt auf die ihn benetzende Nikotinlösung 16 eine Kapillarkraft aus, welche bewirkt, daß der Kapillarspalt 37 rasch geflutet wird. Die Nikotinlösung 16 erreicht den Verdampfer 22 (siehe Fig. 11). Der Verdampfer 22 besteht aus einem Docht und einem elektrischen Heizelement, welche zusammen einen flächigen Verbund bilden. Die Kapillarkräfte im Docht bewirken, daß dieser ebenfalls rasch von der Nikotinlösung 16 infiltriert wird. Gleichzeitig wird auch der aus schlitzförmigen Kapillaren 50 bestehende Pufferspeicher 49 von der Nikotinlösung 16 geflutet. Der Pufferspeicher 49 ermöglicht einen lageunabhängigen Betrieb des Inhalators. Die Dauer zwischen dem Öffnen des Flüssigkeitsbehälters 4 bis zur vollständigen Infiltration des Dochts entspricht einer verbindlichen Wartezeit für den Benutzer und beträgt bei entsprechender Auslegung jedenfalls weniger als 10sec. Der Inhalator ist nun betriebsbereit. Der Benutzer führt über das Mundstück 5 einen Zug ähnlich wie bei einer Zigarette aus. Der Drucksensor 84 (Fig. 8 und Fig 18) detektiert den Beginn des Zuges und veranlaßt den integrierten Schaltkreis 88 das Heizelement des Verdampfers 22 nach einer vorgegebenen Regelungsstrategie mit elektrischer Energie zu versorgen. Dies führt dazu, daß der Verdampfer 22 blitzartig aufgeheizt wird, und die Nikotinlösung 16 im Docht verdampft. Der gebildete Dampf verläßt den Verdampfer 22 über die in weiten Bereichen des Verdampfers freiliegende Dochtoberfläche und mischt sich in der Kammer 21 mit der durch die Lufteinlaßöffnung 26 in die Kammer 21 einströmenden Luft. Durch die Mischung mit der Luft kühlt der Dampf ab und bildet ein Kondensationsaerosol (Fig. 9-10). Überschüssiges Kondensat, welches nicht zur Bildung des Kondensationsaerosols oder Dampf-Luft-Gemisches beiträgt, wird erfindungsgemäß von in der Kammer 21 angeordneten Schwämmen 53 aufgesaugt und gebunden. Das gebildete Dampf-Luft-Gemisch bzw. Kondensationsaerosol strömt schließlich noch durch einen von einer Tabakfüllung 57 ausgebildeten Kühler (Fig. 9-10) oder durch einen Flüssigkeitskühler 64 (Fig. 20), bevor es über den Mundstückkanal 62 in die Mundhöhle des Benutzers gelangt. Nach einer Wartezeit von wenigen Sekunden hat die Nikotinlösung 16 den Docht des Verdampfers 22 von neuem vollständig infiltriert, und der Inhalator ist bereit für eine weitere Inhalation. Enthält der Flüssigkeitsbehälter 4 beispielsweise 5mL effektiv nutzbares flüssiges Material 16 (Fig. 21), und beinhaltet das flüssige Material 16 Nikotin in einer Konzentration von typischerweise 1,5 Vol.-%, dann können mit einer solchen Inhalatorkomponente bis zu 750 Züge bzw. Inhalationen ausgeführt werden, wenn pro Inhalation 100µg Nikotin verdampft werden. 750 Züge entsprechen etwa 75 Zigaretten. Werden pro Zug nur 50µg Nikotin verdampft, dann steigert sich die Reichweite auf 1500 Züge bzw. Inhalationen, welcher Wert etwa 7 1/2 Packungen Zigaretten entspricht.

Obwohl der Verdampfer 22 in den Ausführungsbeispielen stets als flächiger Verbund, bestehend aus einem elektrischen Heizelement und einem Docht, ausgebildet ist, ist die Erfindung selbstverständlich nicht auf diese Ausführungsform beschränkt. Der Verdampfer kann vielmehr jede beliebige Ausführungsform aufweisen, solange er eine in der Kammer 21 angeordnete Verdampfungsfläche bzw. Dampfaustrittsfläche aufweist, aus welcher der erzeugte Dampf in die Kammer 21 übertritt. So könnte der Verdampfer beispielsweise auch als Kapillarrohr-Verdampfer gemäß US 6,155,268 (Manabu Takeuchi) oder US 5,743,251 (Tony M. Howell et al.) ausgebildet sein, wobei in diesem Fall die Kapillarrohröffnung die Dampfaustrittsfläche bilden würde. Auch die Versorgung des Verdampfers mit der Nikotinlösung kann beliebig erfolgen und ist nicht auf die in den Ausführungsbeispielen dargestellte Anspeisung über einen Kapillarspalt 37 beschränkt. Schließlich könnte der Verdampfer anstatt mit Ohmscher Wärme auch mit Induktionswärme, Strahlungswärme oder Mikrowellen aufgeheizt werden. Auch nicht-elektrische Wärmequellen können für die Verdampfung der Nikotinlösung genutzt werden. Die unmittelbare Nutzung von chemischer Reaktionswärme sei als Beispiel genannt.

Abschließend sei noch erwähnt, daß durch die zusätzliche Beimischung von Kohlensäure (CO2) in die angesaugte Luft oder in das gebildete Dampf-Luft-Gemisch bzw. Kondensationsaerosol, beispielsweise durch Einspeisung in die Kammer 21, subjektiv keine nennenswerte Verbesserung der organoleptischen Eigenschaften des verabreichten Dampf-Luft-Gemisches bzw. Kondensationsaerosols erzielt werden konnte. Es wurden diesbezüglich umfangreiche labormäßige Versuche mit erfindungsgemäßen Prototypen und variierenden Kohlensäureanteilen durchgeführt. G. Rudolph, B.A.T. Cigarettenfabriken GmbH, hat erstmals eine solche Beimischung von CO2 bei nikotinhaltigen Zigarettenersatzprodukten vorgeschlagen - vgl. "The Influence of CO2 on the Sensory Characteristics of the Favor-System" (1987), http://legacy.library.ucsf.edu/tid/sla51f00.

### Bezugszeichenliste

- 1: Inhalatorteil
- 2: Inhalatorkomponente
- 3: Gehäuse
- 4: Flüssigkeitsbehälter
- 5: Mundstück
- 6: Batteriedeckel
- 7: Schaltkreisdeckel
- 8: Schnapphaken
- 9: Rastnase
- 10: Trägergehäuse
- 11: elektrischer Schaltkreis, Platine
- 12: Energiespeicher; Batterie
- 13: Trennwand
- 14: Flachkontakt
- 15: Fenster
- 16: flüssiges Material; Nikotinlösung
- 17: Füllloch
- 18: öffenbarer Verschluß
- 19: Verschlußdeckel
- 20: Kontaktelement
- 21: Kammer
- 22: Verdampfer; flächiger Verbund
- 23: plattenförmiger Kontakt
- 24: erste Seite des flächigen Verbundes bzw. Verdampfers
- 25: zweite Seite des flächigen Verbundes bzw. Verdampfers
- 26: Lufteinlaßöffnung; schlitzförmiger Kanal
- 27: Plenumkammer
- 28: Strömungsdrossel
- 29: Querkanal
- 30: Speiseöffnung
- 31: Folie; Metallfolie
- 32: Gewebe; Metalldrahtgewebe
- 33: offenporige Faserstruktur; Vlies
- 34: offenporige Sinterstruktur; körniger, faseriger oder flockiger Sinterverbund
- 35: offenporiger Schaum
- 36: Trägerlage
- 37: Kapillarspalt
- 38: Oberteil
- 39: Platte
- 40: Fortsatz
- 41: Reservoir
- 42: Stift
- 43: erstes Ende
- 44: zweites Ende
- 45: Materialschwächung
- 46: Scharnier
- 47: Querschnittserweiterung
- 48: Belüftungskanal
- 49: Pufferspeicher
- 50: Kapillare; Schlitz
- 51: Öffnung
- 52: Belüftungsspalt
- 53: Schwamm
- 54: Strömungskanal
- 55: Wandabschnitt
- 56: Spalt
- 57: Tabakfüllung; Füll- oder Schüttmaterial
- 58: Füllraum
- 59: Lochwand
- 60: erstes Drahtgewebe
- 61: zweites Drahtgewebe
- 62: Mundstückkanal
- 63: Sammelkammer
- 64: Flüssigkeitskühler
- 65: Behälter
- 66: Kühlflüssigkeit
- 67: Gasblasen
- 68: Pufferraum
- 69: semipermeable Membran; Düsenboden
- 70: semipermeable Membran
- 71: Einbuchtung
- 72: Vorkühler
- 73: vorfabrizierte Packung; Tabakstrang
- 74: Hohlzylinder
- 75: Drahtgewebe
- 76: Mundstück-Kühler-Einheit
- 77: Gehäuseöffnung
- 78: Steckkontakt
- 79: Federkontakt
- 80: Draht
- 81: Zentriervorsprung
- 82: Zentrierausnehmung
- 83: Entlüftungsloch
- 84: Drucksensor
- 85: elektrischer Anschluß; Pin
- 86: Bohrung
- 87: Operationsverstärker; Komparator
- 88: integrierter Schaltkreis; Mikroprozessor
- 89: Leistungs-MOSFET
- 90: Ladestecker

## Patentansprüche

1. Inhalatorkomponente für die Bildung eines nikotinhaltigen Dampf-Luftgemisches oder/und Kondensationsaerosols durch Verdampfung einer mittels Ethanol oder/und Wasser hochverdünnten Nikotinlösung, umfassend:
ein Gehäuse (3);
eine im Gehäuse (3) angeordnete Kammer (21);
eine Lufteinlassöffnung (26) für die Zufuhr von Luft aus der Umgebung in die Kammer (21);
einen Verdampfer (22) zur Verdampfung einer Portion der hochverdünnten Nikotinlösung (16) mit einer in der Kammer (21) angeordneten Verdampfungsfläche beziehungsweise Dampfaustrittsfläche, aus welcher der erzeugte Dampf in die Kammer (21) übertritt und sich in der Kammer (21) mit der durch die Lufteinlassöffnung (26) zugeführten Luft mischt, wodurch sich schließlich das nikotinhaltige Dampf-Luftgemisch oder/und Kondensationsaerosol bildet,
**dadurch gekennzeichnet, dass** die Inhalatorkomponente (2) eine Kondensat-Drainage- und Speichereinrichtung umfasst, wobei die Kondensat-Drainage- und Speichereinrichtung einen Schwamm (53) umfasst.

2. Inhalatorkomponente (2) nach Anspruch 1, wobei der Schwamm (53) gebildet ist aus einem Metallschaum oder einem Keramikschaum, aus einem porösen Sinterformkörper, aus einem porösen Füll- oder Schüttmaterial ohne Blähneigung oder aus einem porösen Faserverbund gebildet aus thermisch oder mithilfe eines Bindemittels miteinander verbundenen Natur- oder Chemiefasern.

3. Inhalatorkomponente (2) gemäß einem der vorhergehenden Ansprüche, wobei der Schwamm (53) weitgehend vom Gehäuse (3) umschlossen wird und mit dem Gehäuse (3) untrennbar verbunden ist.

4. Inhalatorkomponente (2) nach einem der vorhergehenden Ansprüche, wobei die Inhalatorkomponente (2) zwei plattenförmige Schwämme umfasst.

5. Inhalatorkomponente (2) nach Anspruch 4, wobei die Schwämme (53) parallel zueinander angeordnet sind.

6. Inhalatorkomponente (2) nach einem der vorhergehenden Ansprüche, wobei der Schwamm Celluloseacetat umfasst.

7. Inhalator (1) umfassend wenigstens einen Energiespeichermechanismus (12), einen elektrischen Schaltkreis (11) und eine Inhalatorkomponente (2) nach einem der Ansprüche 1 bis 6.

## Claims

1. Inhaler component for the formation of a nicotine-containing vapor-air mixture or/and condensation aerosol by vaporization of a nicotine solution highly diluted by means of ethanol or/and water, comprising:
a housing (3);
a chamber (21) arranged in the housing (3);
an air inlet opening (26) for the supply of air from the environment into the chamber (21);
a vaporizer (22) for vaporizing a portion of the highly diluted nicotine solution (16) with a vaporization surface or vapor exit surface arranged in the chamber (21), from which the generated vapor passes into the chamber (21) and mixes in the chamber (21) with the air supplied through the air inlet opening (26), whereby the nicotine-containing vapor-air mixture or/and condensation aerosol is finally formed,
**characterized in that** the inhaler component (2) comprises a condensate drainage and storage means, wherein the condensate drainage and storage means comprises a sponge (53).

2. The inhaler component (2) according to claim 1, wherein the sponge (53) is formed from a metal foam or a ceramic foam, from a porous sintered molded body, from a porous filler or bulk material without tendency to expand, or from a porous fiber composite formed from natural or chemical fibers bonded to one another thermally or with the aid of a binder.

3. Inhaler component (2) according to any one of the preceding claims, wherein the sponge (53) is largely enclosed by the housing (3) and is inseparably connected to the housing (3).

4. Inhaler component (2) according to any one of the preceding claims, wherein the inhaler component (2) comprises two plate-shaped sponges.

5. Inhaler component (2) according to claim 4, wherein the sponges (53) are arranged parallel to each other.

6. Inhaler component (2) according to any one of the preceding claims, wherein the sponge comprises cellulose acetate.

7. Inhaler (1) comprising at least one energy storage mechanism (12), an electrical circuit (11) and an inhaler component (2) according to any one of claims 1 to 6.

## Revendications

1. Composant d'inhalateur pour la formation d'un mélange vapeur-air ou/et d'un aérosol de condensation contenant de la nicotine par vaporisation d'une solution de nicotine hautement diluée au moyen d'éthanol ou/et d'eau, comprenant :
un boîtier (3) ;
une chambre (21) disposée dans le boîtier (3) ;
une ouverture d'admission d'air (26) pour l'amenée d'air de l'environnement dans la chambre (21) ;
un évaporateur (22) pour l'évaporation d'une portion de la solution de nicotine hautement diluée (16) avec une surface d'évaporation ou une surface de sortie de vapeur disposée dans la chambre (21), à partir de laquelle la vapeur générée passe dans la chambre (21) et se mélange dans la chambre (21) avec l'air amené par l'ouverture d'entrée d'air (26), ce qui forme finalement le mélange vapeur-air ou/et l'aérosol de condensation contenant de la nicotine,
**caractérisé en ce que** le composant inhalateur (2) comprend un dispositif de drainage et de stockage de condensat, le dispositif de drainage et de stockage de condensat comprenant une éponge (53).

2. Composant d'inhalateur (2) selon la revendication 1, dans lequel l'éponge (53) est formée d'une mousse métallique ou d'une mousse céramique, d'un corps moulé fritté poreux, d'un matériau de remplissage ou de vrac poreux sans tendance au gonflement ou d'un composite fibreux poreux formé de fibres naturelles ou chimiques liées entre elles par voie thermique ou à l'aide d'un liant.

3. Composant d'inhalateur (2) selon l'une quelconque des revendications précédentes, dans lequel l'éponge (53) est largement entourée par le boîtier (3) et est inséparable du boîtier (3).

4. Composant d'inhalateur (2) selon l'une quelconque des revendications précédentes, dans lequel le composant d'inhalateur (2) comprend deux éponges en forme de plaque.

5. Composant d'inhalateur (2) selon la revendication 4, dans lequel les éponges (53) sont disposées parallèlement l'une à l'autre.

6. Composant d'inhalateur (2) selon l'une quelconque des revendications précédentes, dans lequel l'éponge comprend de l'acétate de cellulose.

7. Inhalateur (1) comprenant au moins un mécanisme de stockage d'énergie (12), un circuit électrique (11) et un composant d'inhalateur (2) selon l'une quelconque des revendications 1 à 6.
